(19) Europäisches Patentamt
European Patent Office
Office européen des brevets

(11) **EP 4 485 465 A1**

(12) **EUROPEAN PATENT APPLICATION**

(43) Date of publication:
**01.01.2025 Bulletin 2025/01**

(21) Application number: **24184272.3**

(22) Date of filing: **25.06.2024**

(51) International Patent Classification (IPC):
**G16B 30/00** (2019.01)    **G16B 20/50** (2019.01)

(52) Cooperative Patent Classification (CPC):
**G16B 30/00; G16B 20/50**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.06.2023 JP 2023108392**

(71) Applicants:
• **National Cancer Center**
  **Tokyo 104-0045 (JP)**
• **SYSMEX CORPORATION**
  **Kobe-shi**
  **Hyogo 651-0073 (JP)**

(72) Inventors:
• **KOHNO, Takashi**
  **Tokyo, 104-0045 (JP)**
• **SUNAMI, Kuniko**
  **Tokyo, 104-0045 (JP)**
• **ICHIKAWA, Hitoshi**
  **Tokyo, 104-0045 (JP)**
• **SHIOTA, Asuka**
  **Kobe-shi, Hyogo 651-0073 (JP)**
• **FUJIWARA, Kentaro**
  **Kobe-shi, Hyogo 651-0073 (JP)**
• **YOSHIMOTO, Michiko**
  **Kobe-shi, Hyogo 651-0073 (JP)**
• **YUMURA, Mio**
  **Kobe-shi, Hyogo, 651-0073 (JP)**
• **WASHIO, Takanori**
  **Tokyo, 141-0032 (JP)**

(74) Representative: **Hoffmann Eitle**
**Patent- und Rechtsanwälte PartmbB**
**Arabellastraße 30**
**81925 München (DE)**

(54) **METHOD FOR CONTROLLING INFORMATION PROCESSING APPARATUS, INFORMATION PROCESSING APPARATUS, AND COMPUTER PROGRAM**

(57)    Disclosed is a method for controlling an information processing apparatus configured to analyze a sequence of nucleic acid obtained from a specimen of a subject, the method including: obtaining, on the basis of a plurality of first reads read by a sequencer, a result of mutation detection in an analysis target region in the sequence of the nucleic acid; generating, on the basis of second reads, out of the first reads, that correspond to at least one predetermined site selected from a base sequence in the analysis target region, information regarding a quality of a nucleic acid analysis with respect to the predetermined site; and outputting the information regarding the quality, irrespective of presence or absence of a mutation at the predetermined site.

FIG. 4

START
READ OUT READS — S21
OBTAIN RESULT OF MUTATION DETECTION IN ANALYSIS TARGET REGION — S22
GENERATE INFORMATION REGARDING QUALITY OF NUCLEIC ACID ANALYSIS WITH RESPECT TO PREDETERMINED SITE — S23
OUTPUT TEST RESULT REPORT — S24
STORE TEST RESULT REPORT INTO STORAGE UNIT — S25
END

EP 4 485 465 A1

**Description**

CROSS REFERENCE TO RELATED APPLICATIONS

This application claims priority from prior Japanese Patent Application No. 2023-108392, filed on June 30, 2023, entitled "METHOD FOR CONTROLLING INFORMATION PROCESSING APPARATUS, INFORMATION PROCESSING APPARATUS, AND COMPUTER PROGRAM", the entire content of which is incorporated herein by reference.

FIELD OF THE INVENTION

The present invention relates to a method for controlling an information processing apparatus that analyzes a sequence of nucleic acid obtained from a specimen of a subject. The present invention relates to an information processing apparatus that analyzes a sequence of nucleic acid obtained from a specimen of a subject. The present invention relates to a computer program for analyzing a sequence of nucleic acid obtained from a specimen of a subject.

BACKGROUND OF THE INVENTION

In recent years, due to advancement of nucleic acid analysis technologies by next-generation sequencers (NGS), target sequence analysis of analyzing a predetermined region in a genome has become able to be executed at a high throughput. In addition, whole-exome analysis and whole-genome analysis have also become able to be realized at lower costs than before. In the whole-exome analysis, base sequences of all exon regions are analyzed, and in the whole-genome analysis, the sequence of the entire genome is analyzed. The nucleic acid analysis technology by an NGS is also used in clinical genetic diagnosis, and for example, cancer gene panel testing is known. In the cancer gene panel testing, the sequence of nucleic acid obtained from a patient specimen is analyzed by an NGS to detect a gene mutation. A report on a detected gene mutation is created, and a medical worker determines a therapeutic strategy on the basis of the report.

For example, OncoGuide (trademark) NCC Oncopanel System, Report Utilization Guide (https://products.sysmex.co.jp/products/genetic/AK401170/report_guide.pdf) describes an example of an RG sequencing report in which a detected gene mutation is described. In the RG sequencing report, a detected gene mutation and the variant allele frequency with respect to the gene mutation is described. The variant allele frequency is calculated by "mutation depth/total depth". In the RG sequencing report, the values of the mutation depth and the total depth are also described together with the variant allele frequency. The mutation depth is, when a certain base on a reference sequence is focused on, the number of reads that are aligned to the portion including the base and that have a mutation at the position corresponding to the base. The total depth is the number of reads aligned to the portion including the base. The total depth can be used for confirmation of the quality of nucleic acid analysis at the position on the sequence where a mutation has occurred. In the RG sequencing report, the distribution of depth with respect to each gene in the analysis target region is described.

SUMMARY OF THE INVENTION

Medical workers who determine therapies have expressed a demand that they wish to confirm the quality of nucleic acid analysis such as the depth, the quality score, and the like, also with respect to positions on the base sequence where no mutation has been detected. For example, there is a demand, when no mutation has been detected in a gene related to a disease or a therapy, to confirm the quality of nucleic acid analysis with respect to the position. The present invention provides a method for controlling an information processing apparatus, an information processing apparatus, and a computer program that can meet such a demand from medical workers.

The present invention provides a method for controlling an information processing apparatus configured to analyze a sequence of nucleic acid obtained from a specimen of a subject. The method includes: obtaining, on the basis of a plurality of first reads read by a sequencer, a result of mutation detection in an analysis target region in the sequence of the nucleic acid; generating, on the basis of second reads, out of the first reads, that correspond to at least one predetermined site selected from a base sequence in the analysis target region, information regarding a quality of a nucleic acid analysis with respect to the predetermined site; and outputting the information regarding the quality, irrespective of presence or absence of a mutation at the predetermined site.

The present invention provides an information processing apparatus configured to analyze a sequence of nucleic acid obtained from a specimen of a subject. The information processing apparatus includes a controller and an output unit. The controller is configured to execute: obtaining, on the basis of a plurality of first reads read by a sequencer, a result of mutation detection in an analysis target region in the sequence of the nucleic acid; generating, on the basis of second reads, out of the first reads, that correspond to at least one predetermined site selected from a base sequence in the analysis target region, information regarding a quality of a nucleic acid analysis with respect to the predetermined site; and outputting, to the output unit, the information regarding the quality, irrespective of presence or absence of a mutation at the predetermined site.

The present invention provides a computer program for analyzing a sequence of nucleic acid obtained from a specimen of a subject. The computer program is stored in a computer-readable medium and causes the computer to execute: obtaining, on the basis of a plurality of first reads read by a sequencer, a result of mutation detection in an analysis target region in the sequence of the nucleic acid; generating, on the basis of second reads, out of the first reads, that correspond to at least one predetermined site selected from a base sequence in the analysis target region, information regarding a quality of a nucleic acid analysis with respect to the predetermined site; and outputting the information regarding the quality, irrespective of presence or absence of a mutation at the predetermined site.

According to the present invention, even when no mutation has been detected on the base sequence in the analysis target region, the quality of the nucleic acid analysis with respect to the predetermined site of the sequence can be confirmed.

BRIEF DESCRIPTION OF THE DRAWINGS

FIG. 1 shows an example of a configuration of a nucleic acid analysis system;
FIG. 2A shows an example of a configuration of an information processing apparatus;
FIG. 2B shows an example of a configuration of an analysis result database;
FIG. 2C shows an example of a configuration of a sequencer;
FIG. 3 is a flowchart showing an example of a process of reading, by a sequencer, sequences of nucleic acid obtained from a specimen of a subject;
FIG. 4 is a flowchart showing an example of an analysis process on nucleic acid sequence data performed by the information processing apparatus;
FIG. 5 is a flowchart showing an example of a process of detecting a somatic cell mutation and a germ cell mutation by a matched pair test;
FIG. 6A is a table showing examples of companion diagnostics (CDx) markers approved by the US Food and Drug Administration (FDA);
FIG. 6B is a table showing examples of the CDx markers approved by the FDA;
FIG. 6C is a table showing examples of the CDx markers approved by the FDA;
FIG. 7A shows a relationship between first reads mapped to an analysis target region and second reads corresponding to a predetermined site (two places) composed of one base;
FIG. 7B shows a relationship between the first reads mapped to the analysis target region and the second reads corresponding to the predetermined site composed of a plurality of bases;
FIG. 8 is a flowchart showing an example of a process of obtaining information regarding the quality of a nucleic acid analysis with respect to the predetermined site on the basis of each of tumor sequence data and normal sequence data;
FIG. 9 shows an example of a test result report;
FIG. 10 is a flowchart showing an example of a process of determining the predetermined site by a user of the information processing apparatus;
FIG. 11A shows an example of a screen for selecting the predetermined site by inputting category information;
FIG. 11B shows an example of a screen for selecting the predetermined site by inputting category information and subcategory information;
FIG. 11C shows an example of a screen for selecting the predetermined site by inputting category information and subcategory information;
FIG. 11D shows an example of a screen for the user to discretionarily select the predetermined site;
FIG. 12 is a flowchart showing an example of a process of detecting a mutation without the matched pair test; and
FIG. 13 is a flowchart showing an example of a process of obtaining information regarding the quality of the nucleic acid analysis with respect to the predetermined site on the basis of the tumor sequence data.

DETAILED DESCRIPTION

(Nucleic acid analysis system)

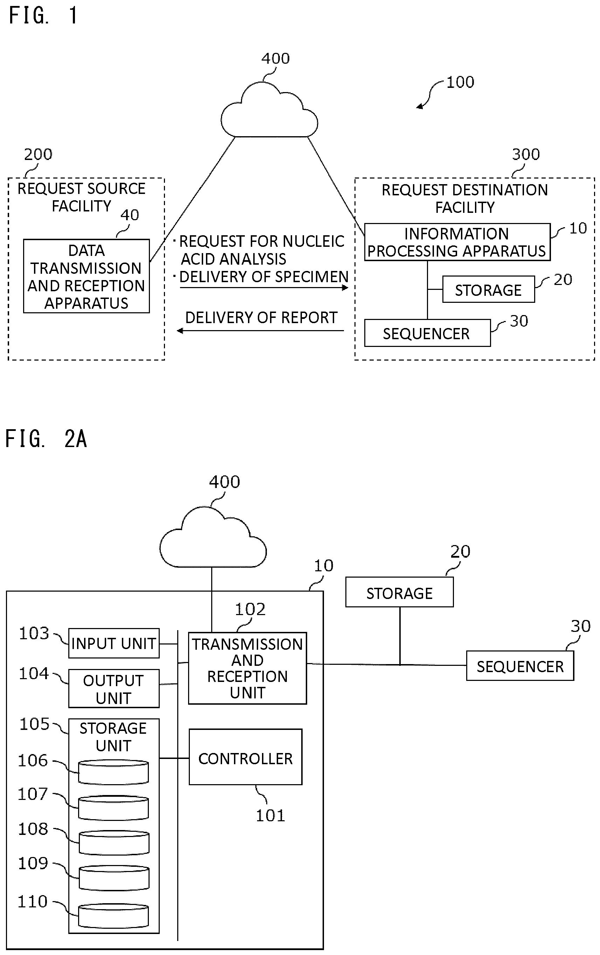

First, a nucleic acid analysis system using an information processing apparatus of the present embodiment will be described. With reference to FIG. 1, a nucleic acid analysis system 100 includes: a data transmission and reception apparatus 40 installed in a request source facility 200 which requests a nucleic acid analysis; and an information processing apparatus 10, a storage 20, and a sequencer 30 which are installed in a request destination facility 300 which performs the nucleic acid analysis in response to a request received from the request source facility 200. The data transmission and reception apparatus 40 and the information processing apparatus 10 are communicably connected to each other via a network 400. In the request destination facility 300, the information processing apparatus 10, the storage 20, and the sequencer 30 are communicably connected to each other via an in-facility network. The data transmission and reception apparatus 40 can be implemented by a computer, for example.

The request source facility 200 is a medical facility, for example. The medical facility is a facility in which medical activities such as collection of a specimen, tests, diagnosis, and treatment are performed on a patient or a person suspected to have a disease, by medical workers such as doctors, nurses, and clinical laboratory technicians. Examples of the medical facility include a hospital, a clinic, a sanatorium, and the like. In the example in FIG. 1, the request source facility 200 does not have a sequencer installed therein, and thus, cannot perform a nucleic acid analysis using a sequencer, such as gene panel testing. Therefore, the request source facility 200 requests a nucleic acid analysis to the request destination facility 300. The request of the nucleic acid analysis is performed through transmission of electronic data (e.g., PDF file) of a request form and/or delivery of the request form printed on paper. In addition, the request source facility 200 delivers a specimen of a subject to the request destination facility 300.

The request destination facility 300 is a facility that performs a nucleic acid analysis in response to the request from the request source facility 200, and provides a test result report describing the analysis result, to the request source facility 200. The request destination facility 300 is a test center, for example. The request destination facility 300 having received the request obtains and analyzes nucleic acid from the specimen provided from the request source facility 200, creates a test result report on the basis of the analysis result, and provides the test result report to the request source facility 200. Provision of the test result report is performed through transmission of electronic data (e.g., PDF file) of the test result report and/or delivery of the report printed on paper.

As shown in FIG. 1, the information processing apparatus 10, the storage 20, and the sequencer 30 of the present embodiment are installed in the request destination facility 300. The information processing apparatus 10 of the present embodiment will be described later. The storage 20 is a device that stores nucleic acid sequence data read by the sequencer 30. The storage 20 can be a network attached storage (NAS), for example. The NAS includes, for example, a storage device such as a hard disk drive (HDD) or a solid-state drive (SSD), a LAN interface, and a simplified OS. In the storage 20, data of a reference sequence described later may be stored in advance.

Herein, "nucleic acid sequence data" denotes reads obtained from a library sample derived from a specimen of a subject. The nucleic acid sequence data encompasses tumor sequence data and normal sequence data described later. "Read" denotes a base sequence of an amplicon read by a sequencer. The library sample, the amplicon, and the read will be described later.

The sequencer 30 is an apparatus for reading base sequences of polynucleotide. Herein, the term "base sequence" is synonymous with "nucleic acid sequence" and "nucleotide sequence". The base sequence denotes one-dimensional arrangement (ordination) of nucleotides in a nucleic acid molecule. Hereinafter, the base sequence may be simply referred to as "sequence". Preferably, the sequencer 30 is a next-generation sequencer. The term "next-generation sequencer" is a term that is used in contrast to "first-generation sequencer", which is a sequencer based on capillary electrophoresis using Sanger's method. The next-generation sequencer can process several-ten million to several-hundred million nucleic acid fragments simultaneously and in parallel, to read sequences. The next-generation sequencer itself is well known, and examples thereof include HiSeq 2500 (Illumina, Inc.), MiSeq (Illumina, Inc.), NextSeq (Illumina, Inc.), Ion Proton (Thermo Fisher Scientific Inc.), Ion PGM (Thermo Fisher Scientific Inc.), GS FLX+ (Roche), GS Junior (Roche), and the like. Data of base sequences read by the sequencer 30 is stored into the storage 20.

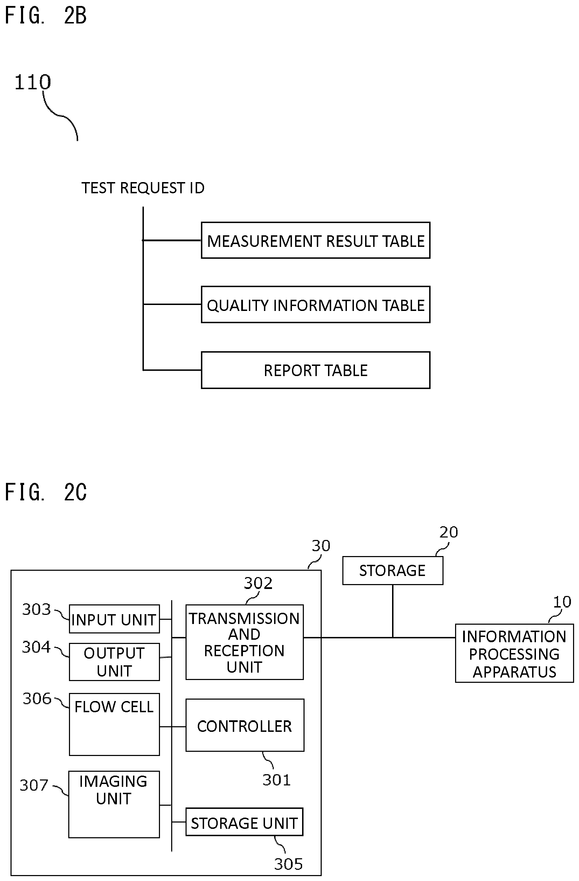

With reference to FIG. 2C, a configuration example of the sequencer 30 will be described. The sequencer 30 includes a controller 301, a transmission and reception unit 302, an input unit 303, an output unit 304, a storage unit 305, a flow cell 306, and an imaging unit 307. The controller 301 includes a processor such as a CPU, and a memory such as a ROM or a RAM, for example. The transmission and reception unit 302 is a communication interface for the controller 301 to communicate with an external apparatus. The input unit 303 is a keyboard, a mouse, a touch sensor, or the like, for example. The output unit 304 is a display, a printer, a speaker, or the like, for example. An apparatus that has functions of both of an input unit and an output unit, such as a touch panel in which a touch sensor and a display are integrated, may be used. The storage unit 305 is a storage device such as an HDD or an SSD, for example. The flow cell 306 includes a glass substrate having a flow path in which a reagent flows. The flow cell 306 allows formation of a cluster and sequence reaction of polynucleotide included in a library sample introduced in the flow path. The imaging unit 307 captures an image of the cluster in the flow cell 306, and stores the captured image into the storage unit 305. The controller 301 analyzes the image stored in the storage unit 305 and determines the base sequence of the polynucleotide.

"Library" means a collection of amplicons (amplification product) of which the base sequences are to be analyzed by a sequencer. "Library sample" is a sample including a library, and is prepared by amplifying nucleic acid obtained from the specimen. In preparation of a library sample, first, nucleic acid is extracted from a specimen of a subject. The type of the nucleic acid is selected from DNA and RNA in accordance with the purpose of the analysis. In extraction of DNA, a specimen and a lysate including a surfactant (e.g., sodium cholate, sodium dodecyl sulfate) that causes lysis of a cell or a tissue are mixed together. Physical processing (agitation, homogenization, ultrasonic crushing, etc.) is performed on the obtained mixture, to release DNA included in the specimen into the liquid. In extraction of RNA, a specimen and a lysate including guanidine thiocyanate and a surfactant are mixed together. Physical processing is performed on the obtained mixture, to release RNA included in the specimen into the liquid.

When the specimen is a formalin-fixed paraffin-embedded (FFPE) tissue, extraction of nucleic acid can be performed as follows, for example. First, xylene is added to the FFPE tissue, to be subjected to deparaffinization. The deparaffinized tissue is soaked into ethanol, to be subjected to hydrophilization. The hydrophilized tissue is processed by a protease, to release nucleic acid cross-linked with formalin, into the liquid. Preferably, the mixture including DNA or RNA is subjected to centrifugation or the like to remove cell fragments, to obtain a solution including the released DNA or RNA. Then, the obtained solution is subjected to phenol/chloroform extraction, whereby DNA or RNA can be purified. Extraction and purification of nucleic acid from the specimen may be performed using a commercially available reagent kit.

In preparation of a library sample, it is preferable to fragment the nucleic acid. Fragmentation allows the obtained nucleic acid to have a length (several tens to several hundred bps) suitable for reading by the sequencer 30. The nucleic acid can be fragmented by ultrasonic processing, for example. When the nucleic acid is DNA, fragmentation can also be performed by alkali treatment, restriction enzyme treatment, or the like. For example, when DNA is fragmented by alkali treatment, if a sodium hydroxide solution is added into a DNA solution so as to have a final concentration of 0.1 to 1.0 N, and the resultant mixture is incubated at 10 to 40°C for 5 to 15 minutes, DNA is fragmented. When DNA is fragmented by a restriction enzyme treatment, the restriction enzyme is selected as appropriate on the basis of the base sequence of DNA, and Msel, BamHI, or the like is used, for example. Size selection of nucleic acid fragments, terminal smoothing, ligation of an adaptor sequence, ligation of an index sequence, ligation of a barcode sequence, or the like may be performed.

Preferably, the fragmented nucleic acid is amplified by a method based on PCR. When a primer set that can amplify the analysis target region is designed, and by using it, nucleic acid is amplified by a PCR method, a library sample can be obtained. One primer set includes one forward primer and one reverse primer. Nucleic acid including the analysis target region may be enriched from nucleic acid fragments by a sequence capture method. When amplification is performed by using the enriched nucleic acid as the template for the PCR method, a library sample can be obtained. Hereinafter, a library sample derived from DNA in a specimen will also be referred to as "DNA library sample".

An addition sequence such as an adaptor sequence, an index sequence, or a barcode sequence, a labeling substance, or the like may be added to the primer used in amplification. When a plurality of primer sets are used, these primer sets are preferably able to be used in multiplex PCR. Accordingly, a plurality of regions in the obtained nucleic acid can be simultaneously amplified. In this case, it is preferable that barcode sequences different from each other are added to the respective primer sets. Accordingly, an amplicon according to each primer set can be identified. A multiplex PCR primer set attached to a commercially available reagent kit such as an exome sequencing kit may be used.

A polymerase used in amplification can be selected as appropriate from known heat resistant polymerases used in PCR. Among them, a heat resistant polymerase suitable for multiplex PCR and having few errors due to PCR amplification is desirable. For amplification reaction, a buffer suitable for the selected polymerase may be used. In order to suppress errors due to PCR amplification, it is preferable that the cycle number of PCR is set to be minimum within a range in which a necessary number of amplicons for sequencing can be obtained. The cycle number may be determined in a range of 10 cycles or more and 25 cycles or less, for example.

With respect to the library sample obtained as above, base sequences may be read according to a sequencing method known in the technical field. The sequencing method is not limited in particular, but an analysis by a next-generation sequencer is preferable. Examples of the analysis method by the next-generation sequencer include an ion semiconductor sequencing method, a pyrosequencing method, and an SBS (sequencing by synthesis) method, and the like.

The information processing apparatus 10 of the present embodiment is an apparatus for analyzing the sequence of nucleic acid obtained from a specimen of a subject. With reference to FIG. 2A, an example of a configuration of the information processing apparatus 10 will be described. The information processing apparatus 10 includes a controller 101, a transmission and reception unit 102, an input unit 103, an output unit 104, and a storage unit 105. The storage unit 105 has stored therein a reference sequence database 106, a mutation information database 107, an analysis target table 108, a quality index table 109, and an analysis result database 110. Alternatively, the reference sequence database 106, the mutation information database 107, the analysis target table 108, the quality index table 109, and the analysis result database 110 may be stored in the storage 20. Information included in each database/table will be described later. The information processing apparatus 10 can be implemented by a computer, for example. In this case, the controller 101 includes a processor such as a CPU, and a memory such as a ROM or a RAM, for example. The transmission and reception unit 102 is a communication interface for the controller 101 to communicate with an external apparatus and the network 400. The input unit 103 is a keyboard, a mouse, a touch sensor, or the like, for example. The output unit 104 is a display, a printer, a speaker, or the like, for example. An apparatus that has functions of both of an input unit and an output unit, such as a touch panel in which a touch sensor and a display are integrated, may be used. The storage unit 105 is a storage device such as an HDD or an SSD, for example. In the storage unit 105, a computer program for analyzing sequences of nucleic acid is stored. The controller 101 reads out the computer program from the storage unit 105 and executes an analysis process on nucleic acid sequences described later.

Information included in the reference sequence database 106 and the mutation information database 107 can be periodically updated to newest information on the basis of information of an external database. Update of the information may be performed by a user or a vendor that provides the nucleic acid analysis system. Alternatively, update of information may be performed automatically by the nucleic acid analysis system. Examples of the external database include a database in which information (information of reference sequences, and various types of information regarding known gene mutations) provided from public institutions and academies of countries is accumulated, a commercial database provided from a vendor, and the like.

"Reference sequence" is a sequence to serve as a reference for determining where on the genome region a read, which is a base sequence, corresponds to. The reference sequence may be any sequence including the base sequence in the analysis target region. Examples of the reference sequence include the base sequence of the whole-genome region of human, the base sequence of the whole-exon region, the base sequence of a target gene, and the like.

"Mutation" and "gene mutation" encompass spontaneous mutation and genetic polymorphism. Examples of mutation include sequence variation, structural variation, and copy number variation. Sequence variation is substitution, insertion, or deletion of one or a plurality of nucleotides, and a combination thereof. Examples of substitution of a nucleotide include single nucleotide variation (SNV) and single nucleotide polymorphism (SNP). Insertion and deletion of a nucleotide is also referred to as "InDel". Examples of structural variation include translocation, inversion, deletion, and duplication. Examples of copy number variation include amplification and deletion.

The analysis target table 108 includes information of the analysis target region. The information of the analysis target region describes information of a target gene selected as the analysis target region, for example. Examples of the information of a target gene include the name of the gene, position information, and the base sequence. Examples of the position information include the chromosome number (Chr), and the start point (Pos-Start) and the end point (Pos-End) of the base sequence of the gene on the gene (Gene) genome sequence. Table 1 shows an example of the analysis target table 108, but the analysis target table 108 is not limited thereto.

[Table 1]

| Chr | Gene | Pos_Start | Pos_End |
|---|---|---|---|
| 1 | gene A | Position nnn | Position mmm |
| 1 | gene A | ... | ... |
| 2 | gene B | ... | ... |
| 2 | gene B | ... | ... |
| 2 | gene B | ... | ... |
| 7 | gene C | ... | ... |
| ⋮ | ⋮ | ⋮ | ⋮ |

"Analysis target region" is a region for which analysis of the base sequence in the nucleic acid obtained from a specimen of a subject is desired. The analysis target region can be discretionarily determined from a genome region. Preferably, the analysis target region is a part of a genome region including at least one target gene. The analysis target region may be the whole-genome region or the whole-exome region. The number of the analysis target regions may be one or may be a plurality. For example, in gene panel testing, a nucleic acid analysis is performed using several tens or hundreds or more of genes as the analysis target region.

"Target gene" is a gene for which determination of the presence or absence of a mutation is desired. The target gene is not limited in particular, and can be determined as appropriate in accordance with the purpose of the test. Examples of the target gene include genes that are used in known gene panel testing. The base sequence of a target gene encompasses base sequences in the transcription regulation regions of the exon, the intron, the promoter, and the like of the gene, and the sequence of mRNA transcribed from the gene. mRNA encompasses pre-mRNA.

The nucleic acid analysis system 100 can be used when gene panel testing is performed, for example. Gene panel testing is not limited to clinical tests and also encompasses tests for research use. For example, the request source facility 200 transmits request information on a nucleic acid analysis such as gene panel testing from the data transmission and reception apparatus 40 to the request destination facility 300. In addition, the request source facility 200 delivers a specimen of the subject to the request destination facility 300.

The subject is not limited in particular, and examples thereof include a patient, a person suspected to have a disease, a healthy individual, and the like. The disease is not limited in particular, and examples thereof include diseases for which a genetic test or genomic medicine is considered to be useful. Examples of such a disease include cancers, autoimmune diseases, genetic diseases, and the like. The specimen is not limited in particular as long as the specimen includes nucleic acid of a subject. Preferably, the specimen is a biological sample collected from a subject. Examples of the biological sample include tissue, cell, body fluid, secretory fluid, urine, stool, and the like. Examples of the body fluid include blood (whole blood), bone marrow aspirate, cerebrospinal fluid, lymph, ascites, pleural effusion, amniotic fluid, synovial fluid, and the like. Examples of the secretory fluid include saliva, sweat, tear fluid, nasal discharge, semen, papillary secretory fluid, and the like. The specimen may be a preparation from a biological sample. Examples of the preparation from blood include plasma and serum. Examples of the preparation from cells include a culture (including cultured cells and medium) obtained by culturing collected cells. Examples of the preparation from a tissue include a frozen tissue, a fixed tissue, an FFPE tissue, and the like.

When a matched pair test is performed on a subject who is a patient of a solid cancer, two types, i.e., a tumor specimen and a non-tumor specimen, are used as the specimen. The matched pair test is a test in which the sequence of nucleic acid obtained from each of a tumor specimen and a non-tumor specimen of a solid cancer patient is analyzed, and a somatic cell mutation and a germ cell mutation can be distinguished to be detected on the basis of the result of the analyses. The tumor specimen can be a tumor tissue collected from a subject through surgery or biopsy, and a preparation thereof, for example. The non-tumor specimen can be non-tumor cells (e.g., whole blood) collected from the same subject.

The solid cancer denotes a cancer other than blood cancers out of cancers. The type of the solid cancer is not limited in particular, and may be either of an epithelial cell cancer or a non-epithelial cell cancer. The epithelial cell cancer is not limited in particular as long as the epithelial cell cancer is a cancer that occurs in epithelial cells, and examples thereof include lung cancer, breast cancer, gastric cancer, colon cancer, liver cancer, uterine cancer, ovarian cancer, and the like. Examples of the non-epithelial cell cancer include osteosarcoma, chondrosarcoma, rhabdomyosarcoma, leiomyosarcoma, fibrosarcoma, liposarcoma, angiosarcoma, and the like.

The quality index table 109 includes at least, as information regarding a predetermined site described later, position information of the predetermined site in the analysis target region. As shown in FIG. 2B, the analysis result database 110 includes, for each test request, a measurement result table including a result of mutation detection in the analysis target region, a quality information table including information regarding the quality of the nucleic acid analysis with respect to the predetermined site, and a report table including information of a test result report generated from those. Each table is associated with a test request ID that individually identifies a test request.

In the following, an analysis of a nucleic acid sequence performed by the nucleic acid analysis system 100 will be described. When a person in charge in the request source facility 200 has inputted request information on a nucleic acid analysis to the data transmission and reception apparatus 40, the request information is transmitted to the information processing apparatus 10 via the network 400, and received by the information processing apparatus 10. The request information may include information of the subject, information of the specimen, and the like, for example. The information of the subject includes the age, the gender, the type and state of a disease, and the like, for example. The information of the specimen includes the type of the specimen, the time of collection, a specimen ID that associates the specimen and the subject, and the like, for example. The request information may include designation of the analysis target region and/or the predetermined site. In the case of a request for gene panel testing, the request information may include designation of a desired gene panel. The predetermined site will be described later. A person in charge in the request destination facility 300 inputs a request for an analysis, to the sequencer 30, in accordance with the request information received by the information processing apparatus 10.

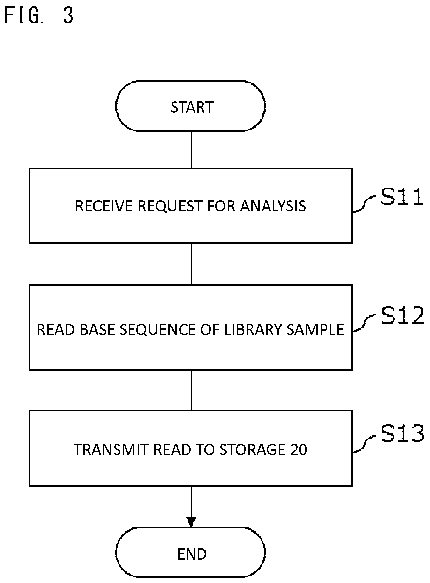

With reference to FIG. 3, reading of base sequences by the sequencer 30 will be described. In step S11, the controller 301 of the sequencer 30 receives a request for an analysis. In step S12, the controller 301 of the sequencer 30 reads base sequences of a library sample.

As described above, the base sequence of an amplicon read by a sequencer is a "read". That is, in step S12, the sequencer 30 reads the base sequence of each amplicon of the library sample, to obtain reads. The length of the read is different according to the analysis method, the type of the sequencer, and the like, and is, for example, 50 bases or more, preferably 100 bases or more, and more preferably 150 bases or more. The length of the read is, for example, 500 bases or less, preferably 350 bases or less, and more preferably 250 bases or less. The number of the reads is not limited in particular, and is different depending on the copy number of the nucleic acid obtained from the specimen, the site at which amplification is performed by the PCR method, and the like.

In step S13, the controller 301 of the sequencer 30 transmits all of the obtained reads to the storage 20. The storage 20 stores those reads. The file format of the reads transmitted from the sequencer 30 is FASTA, FASTQ, or uBAM, for example. A FASTA file is a file including only the sequence information of the reads. A FASTQ file is a file including sequence information of the reads including quality scores described later. A uBAM file is a binary file including sequence information of reads that are not aligned to the reference sequence. The alignment will be described later. In step S13, the controller 301 of the sequencer 30 may transmit the obtained reads to the information processing apparatus 10. In this case, the information processing apparatus 10 stores those reads.

The file that is transmitted from the sequencer 30 may include information such as the name (or sequence ID) of each read and the quality score of each base in the sequence, together with data of the read. The quality score is an index indicating the correctness of base call (designation of bases) by the sequencer. Here, the base sequence of a read is determined by performing base call on trace data (raw data such as waveform data of a signal obtained in sequencing reaction) obtained by the sequencer. That is, the quality score indicates the correctness of the base sequence determined by the sequencer. The base call is executed by a known base calling program such as Phred, for example. As the quality score, for example, a quality score (Q) outputted from a sequencer (HiSeq 2500, MiSeq, NextSeq, or the like) of Illumina, Inc. can be used. The quality score (Q) is calculated by Formula (I) below. In the formula, E is an estimated value of the probability that the base call is wrong.

$$Q = -10 \log_{10} E \ \ldots (I)$$

The quality score is provided to each base of a read. For example, when the quality score of a base in a read is 20, the frequency of error in the base is $10^{-2}$/base. When the quality score is 30, the frequency of error in the base is $10^{-3}$/base. The average value of the quality scores in a read can indicate the frequency of error. The average value of the quality scores can be calculated by dividing the sum of the quality scores of respective bases of a read by the length (the number of bases) of the read. For example, when the average value of the quality scores is 20, the error in the read is one in 100 bases. When the average value of the quality scores is 30, the error in the read is one in 1,000 bases. Thus, the higher the quality score is, the lower the probability of errors is. The quality scores and the average value thereof are automatically calculated by a next-generation sequencer.

When the matched pair test of which the analysis target is DNA is to be performed, a DNA library sample is prepared from each of a tumor specimen (e.g., FFPE specimen of a tumor tissue) and a non-tumor specimen (e.g., whole blood). Then, the sequencer 30 reads, in step S12, the base sequence of each amplicon of each library sample, to obtain reads. In the following, the read obtained from a DNA library sample derived from a tumor specimen will be referred to as "tumor sequence data". In addition, the read obtained from a DNA library sample derived from a non-tumor specimen will be referred to as "normal sequence data".

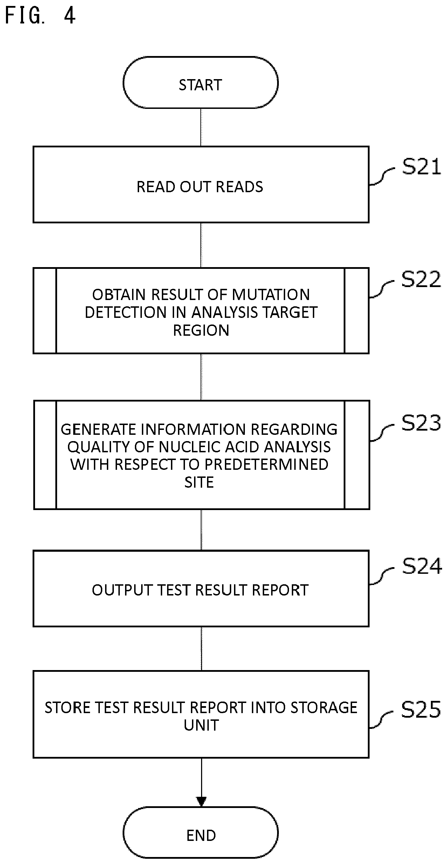

With reference to FIG. 4, an example of an analysis process on the nucleic acid sequence data performed by the information processing apparatus 10 will be described. In step S21, the controller 101 of the information processing apparatus 10 reads out reads stored in the storage 20. In step S22, the controller 101 obtains a result of mutation detection in the analysis target region on the basis of the reads.

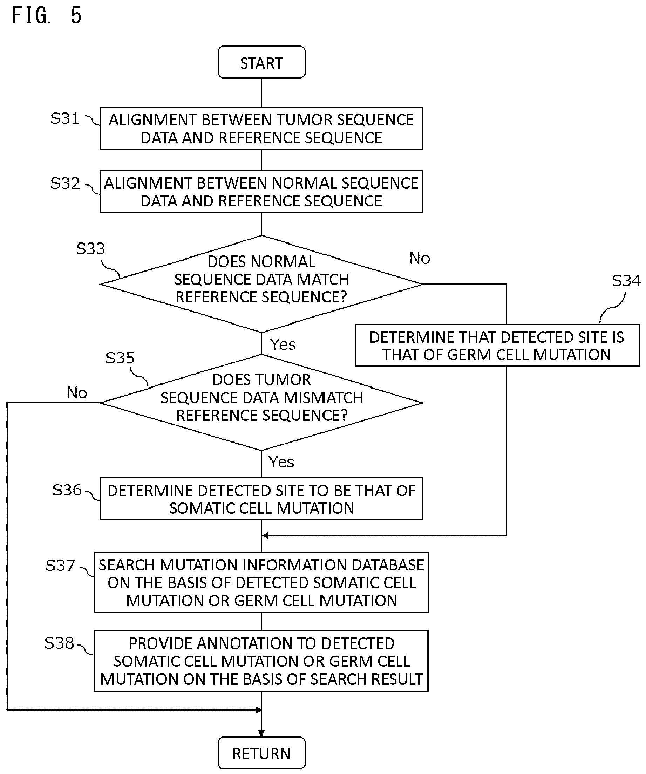

As one embodiment of step S22, a flow of a process of detecting a somatic cell mutation and a germ cell mutation by the matched pair test will be described. The somatic cell mutation is a mutation that has occurred in a gene of a somatic cell. It is known that a somatic cell mutation is one of causes of a tumor. With reference to FIG. 5, in step S31, the controller 101 reads out a reference sequence from the reference sequence database 106, and executes alignment between the tumor sequence data read out in step S21 and the reference sequence. In step S32, the controller 101 executes alignment between the normal sequence data and the reference sequence. The result of the alignment between the tumor sequence data and the reference sequence and the result of the alignment between the normal sequence data and the reference sequence are stored into the storage unit 105.

"Alignment" is a process of determining, with respect to each read, a corresponding region on the reference sequence. Alignment is also referred to as mapping. The reference sequence may be any base sequence that does not include any somatic cell mutation. For example, the reference sequence is a base sequence of a wild type genome. For alignment between the read and the reference sequence, a known alignment tool (software) such as BWA (Burrows-Wheeler Aligner), BWA-MEM, Bowtie, Bowtie2, or BLAST can be used.

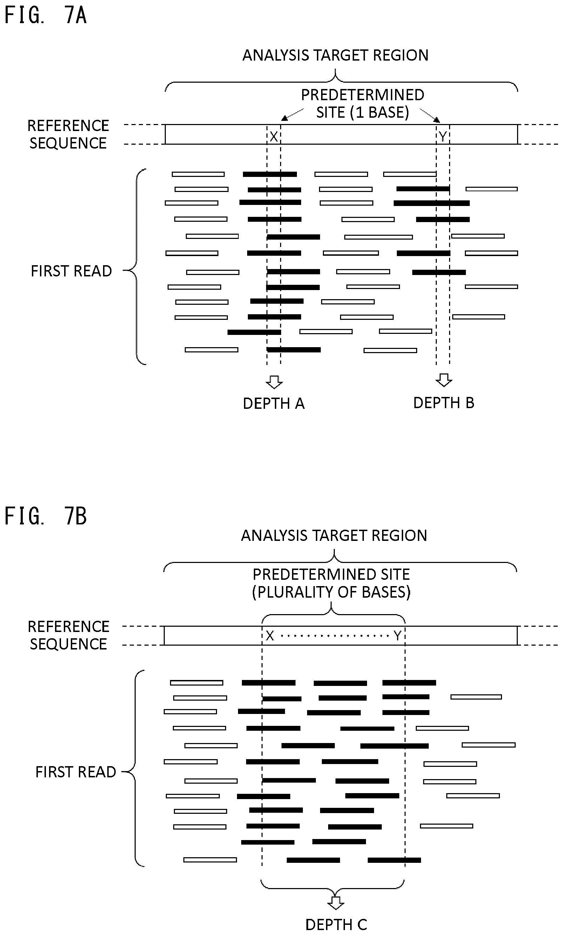

In the following, out of the reads, a read corresponding to the analysis target region will also be referred to as a "first read". A "read corresponding to the analysis target region" is a read, when alignment with respect to the reference sequence including the base sequence of the analysis target region has been executed, that has been placed at the base sequence of the analysis target region. As shown in FIGS. 7A and 7B described later, usually, a plurality of the first reads are mapped to the base sequence of the analysis target region. Usually, the base sequence of the analysis target region is longer than the first read. In the drawing, the first reads are indicated by white bars and black bars. Out of the first reads, the black bars each indicate a second read described later.

The controller 101 may calculate a mapping quality score of each sequence read. The mapping quality score is an index indicating the correctness of mapping of a read. The mapping quality score (Q) is calculated by Formula (II) below. In the formula, P is the probability that the read is mapped at a wrong position.

$$Q = -10\log_{10}P \quad \dots(II)$$

The higher the value of the mapping quality score of a certain read is, the lower the probability that the read is mapped at a wrong position is. Therefore, when the mapping quality score of a read is high, it can be said that the read has a high possibility of having the base sequence corresponding to the analysis target region.

In the tumor sequence data, when there is a site that does not match the reference sequence, the site can be that of a mutation of a gene of the tumor tissue or a genetic polymorphism unique to the subject. In the normal sequence data, when there is a site that does not match the reference sequence, the site can be that of a genetic polymorphism unique to the subject or a germ cell mutation.

In step S33, the controller 101 determines whether or not there is a site that does not match the reference sequence in the normal sequence data. As described above, when there is a site that does not match the reference sequence, the site can be that of a germ cell mutation or a genetic polymorphism unique to the subject. When the controller 101 has determined that the normal sequence data does not match the reference sequence, the process advances to step S34. In step S34, the controller 101 determines the site that does not match the reference sequence in the normal sequence data detected in step S33, to be that of a germ cell mutation. Then, the process advances to step S37 described later. Meanwhile, in step S33, when the controller 101 has determined that the normal sequence data matches the reference sequence, the process advances to step S35. In step S35, the controller 101 determines whether or not the tumor sequence data corresponding to the site that matches the reference sequence in the normal sequence data detected in step 33 matches the reference sequence. When the tumor sequence data does not match the reference sequence, the site that does not match the reference sequence in the tumor sequence data detected in step S35 has a high possibility of being that of a mutation that is observed only in a gene of a tumor tissue. The mutation observed only in a gene of a tumor tissue is a somatic cell mutation. Therefore, in step S36, the controller 101 determines that the site detected in step 35 to be that of a somatic cell mutation.

In step S35, when the tumor sequence data matches the reference sequence, the controller 101 ends the process. In this case, it is considered that the subject has neither a somatic cell mutation nor a germ cell mutation.

In step S37, the controller 101 searches the mutation information database 107 on the basis of the detected somatic cell mutation or germ cell mutation. In the mutation information database 107, information for specifying a known gene mutation is accumulated. Examples of such information include mutation identifier (mutation ID), gene name, position information of the mutation (e.g., chromosome number (Chr) and base number (Pos) in the genome sequence), REF, ALT, annotation, and the like. The mutation ID is an identifier for identifying the mutation. Out of the position information of the mutation, Chr indicates the chromosome number at which the mutation is positioned. Pos indicates the base number in the genome sequence. REF indicates a base in the wild type, and ALT indicates a mutated base. Annotation indicates information of amino acid and/or base that has changed due to the mutation. Annotation may be displayed as "p.A146V", "c.437C>T", or the like, for example. "p.A146V" means that the 146th alanine residue of protein has changed to a valine residue due to the mutation. "c.437C>T" means that the 437th cytosine has changed to a thymine due to the mutation.

For example, on the basis of the name and the position information of the gene where the detected somatic cell mutation or germ cell mutation is present, the controller 101 can search the mutation information database 107. Accordingly, which of the known gene mutations the detected somatic cell mutation or germ cell mutation corresponds to can be determined.

The mutation information database 107 may further store not only the information for specifying known gene mutations but also disease information, drug information, and biomarker attribution information that are related to those known gene mutations. Examples of the disease information include: the disease name (e.g., colon cancer, lung cancer, breast cancer, etc.) related to a known gene mutation; the type of disease (e.g., cancer, eye disease, neuropsychiatric disease, etc.); information regarding driver mutations; information regarding pathogenicity evaluation of the mutation (e.g., pathogenic, likely pathogenic, benign, etc.); information (e.g., evidence level) regarding the relevance level between the mutation and the disease; information of the type of the relevance between the mutation and the disease (e.g., oncogenic evidence (Oncogenic), predisposing evidence (Predisposing), predictive evidence (Predictive), diagnostic evidence (Diagnostic), prognostic evidence (Prognostic)); and the like. Examples of the drug information include information such as the presence or absence of a therapeutic drug related to a known gene mutation, the name of the therapeutic drug, and the country/region where the therapeutic drug has been approved. Examples of the biomarker attribution information include: the type of the biomarker (e.g., companion diagnostics (CDx), malignancy, prognosis, morbidity risk, onset risk, etc.); and information on a marker test (e.g., the disease name to which the test is applied, the name of the therapeutic drug, the country/region where the test has been approved, etc.).

In step S38, the controller 101 provides an annotation to the detected somatic cell mutation or germ cell mutation on the basis of the search result. Through the processes of steps S31 to S3 8, the controller 101 obtains, in step S22, information of the gene mutation in the analysis target region, as the result of mutation detection. Examples of the information of the gene mutation include: the name (Gene) of the gene where the detected mutation has occurred; position information of the mutation (chromosome number (Chr), base number (Pos) in the genome sequence, etc.); amino acid change (AA) in the gene mutation; coding region change (CDS) in the gene mutation; information of the type (Somatic/Germline) of the somatic cell mutation or the germ cell mutation; the type of the mutation (Mutation type); and the like.

The controller 101 stores the result of mutation detection obtained through the process of step S22 into the measurement result table in the analysis result database 110. Table 2 shows an example of the measurement result table, but the measurement result table is not limited thereto. The measurement result table includes all of the gene mutations detected through the process of step S22. In the table, "Chr" indicates the chromosome number of the detected gene mutation, "Pos" indicates the base number in the genome sequence of the detected gene mutation, "CDS" indicates the coding region change in the detected gene mutation, "AA" indicates the amino acid change in the detected gene mutation, "Somatic/Germline" indicates the type, i.e., whether the detected gene mutation is a somatic cell mutation or a germ cell mutation, and "Mutation type" indicates the type of the detected gene mutation.

[Table 2]

| Chr | Gene | Pos | CDS | AA | Somatic/ Germline | Mutation type |
|---|---|---|---|---|---|---|
| 1 | gene A | 115,252,203 | c.437C>T | A146V | somatic | SNV |
| 7 | gene C | ▪ ▪ ▪ | ▪ ▪ ▪ | ▪ ▪ ▪ | ▪ ▪ ▪ | ▪ ▪ ▪ |
| ・・・ | ・・・ | ・・・ | ・・・ | ・・・ | ・・・ | ・・・ |

The controller 101 ends the process of obtaining the result of mutation detection, and the process advances to step S23 in FIG. 4. In step S23, on the basis of the nucleic acid sequence data, the controller 101 generates information regarding the quality of the nucleic acid analysis with respect to the predetermined site.

"Predetermined site" is at least one site discretionarily selected from the base sequence in the analysis target region. The predetermined site is selected in order to examine the quality of the nucleic acid analysis and provide information regarding the quality. Determination of the predetermined site by the controller 101 will be described later. In the base sequence in the analysis target region, the number of the predetermined site may be one or may be a plurality. The predetermined site is composed of one base or two or more consecutive bases in the base sequence in the analysis target region. That is, the predetermined site may be one base or may be a region composed of a plurality of consecutive bases. When the predetermined site is a region composed of a plurality of consecutive bases, the length thereof can be 2 bases, 3 bases, 4 bases, 5 bases, 6 bases, 7 bases, 8 bases, 9 bases, 10 bases, or 11 bases, for example.

Preferably, the predetermined site includes a site having a possibility of occurrence of a mutation. The predetermined site may be a site having a possibility of occurrence of a mutation, or may be a site having a possibility of occurrence of a mutation or the vicinity thereof. For example, with respect to a known mutation in the analysis target region, when it is known that a substitution or a deletion can occur or one base can be inserted at a certain base, the predetermined site may be the one base itself where the mutation can occur. Alternatively, the predetermined site may be a region extending by one to five bases on each of the upstream side and the downstream side with respect to the one base where the mutation can occur. In this case, the predetermined site may be a region including one base where the mutation can occur. It does not matter whether or not a mutation has actually occurred at the predetermined site. As a result of the nucleic acid analysis, a mutation may or may not have occurred at the predetermined site.

The mutation having a possibility of occurring at the predetermined site is a mutation, in the analysis target region, related to diagnosis or therapy of a disease, for example. Examples of such a mutation include mutations described in (1) to (3) below.

(1) a mutation known to be related to an effect and/or a side effect of a drug,
(2) a mutation known to be related to the state, onset, and/or prognosis of a disease, and
(3) a mutation of which the evidence level indicating the relevance to diagnosis or therapy is known to be at a predetermined level or higher.

The mutation described in (1) is a companion diagnostics marker (CDx marker), for example. As such a marker, a gene mutation used in gene panel testing such as FoundationOne (registered trademark) CDx cancer genome profile (Chugai Pharmaceutical Co., Ltd.) is known, for example. The mutation described in (2) is a driver mutation, a pathogenic mutation, a mutation as a malignancy marker, a mutation as a prognostic marker, a mutation as a morbidity risk marker, or a mutation as an onset risk marker, for example. As such a marker, gene mutations used in OncoGuide (trademark) NCC oncopanel system (Sysmex Corporation) and FoundationOne (registered trademark) CDx cancer genome profile (Chugai Pharmaceutical Co., Ltd.) are known, for example.

The evidence level indicates the degree at which the evidence is reliable or recommendable. The evidence level described in (3) is the evidence level of the oncogenic evidence, the predisposing evidence, the predictive evidence, the diagnostic evidence, or the prognostic evidence, for example. The oncogenic evidence denotes a gene mutation, such as a somatic cell mutation or a germ cell mutation, that contributes to malignant transformation of a cell. The predisposing evidence denotes a germ cell mutation related to malignant transformation in inherited breast cancer, ovarian cancer, or the like. The predictive evidence denotes a marker related to sensitivity and resistance with respect to a drug, radiation therapy, or the like, a gene mutation of a drug transporter or a drug metabolism enzyme that influences pharmacokinetics, and the like. The diagnostic evidence is a marker related to diagnosis of a patient, and examples thereof include cell morphology, tumor histology, manifestation or localization of a cancer-type specific marker protein according to immunohistochemical staining, and the like. The prognostic evidence denotes a marker regarding advancement of the cancer, severity, survival prognosis, and the like. The evidence levels of these pieces of evidence have been published from academies of, for example, the Japanese Society of Medical Oncology, the Japan Society of Clinical Oncology, the Japanese Cancer Association, the American Society of Clinical Oncology (ASCO), the College of American Pathologists (CAP), the Association for Molecular Pathology (AMP), the European Society for Medical Oncology (ESMO), and the like.

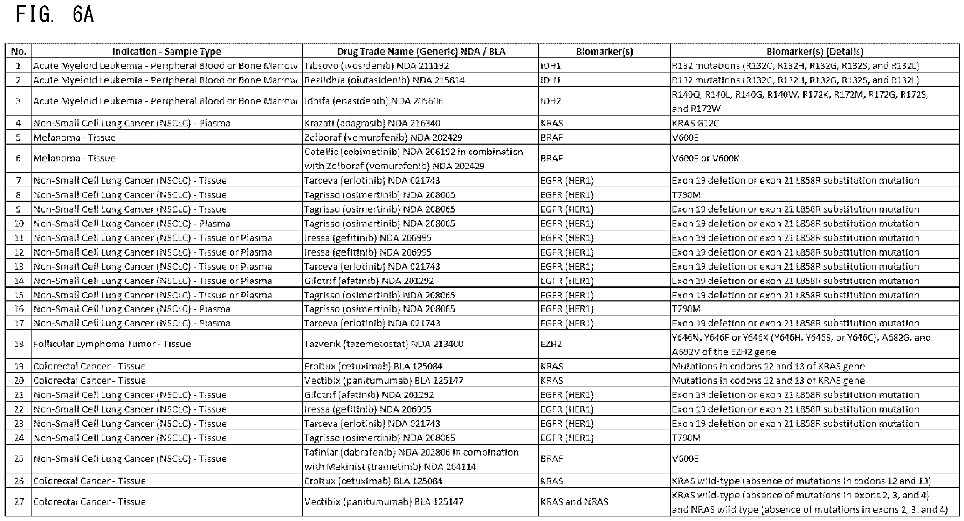

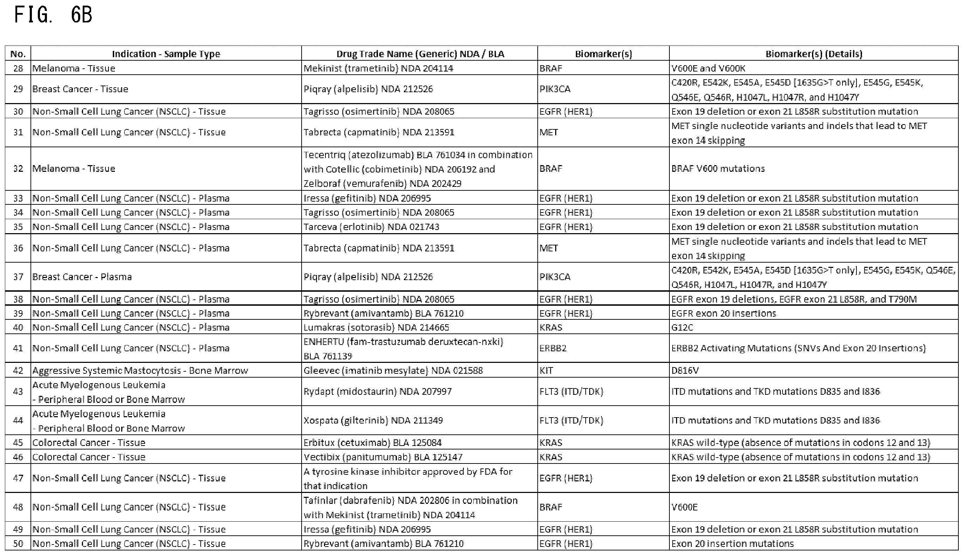

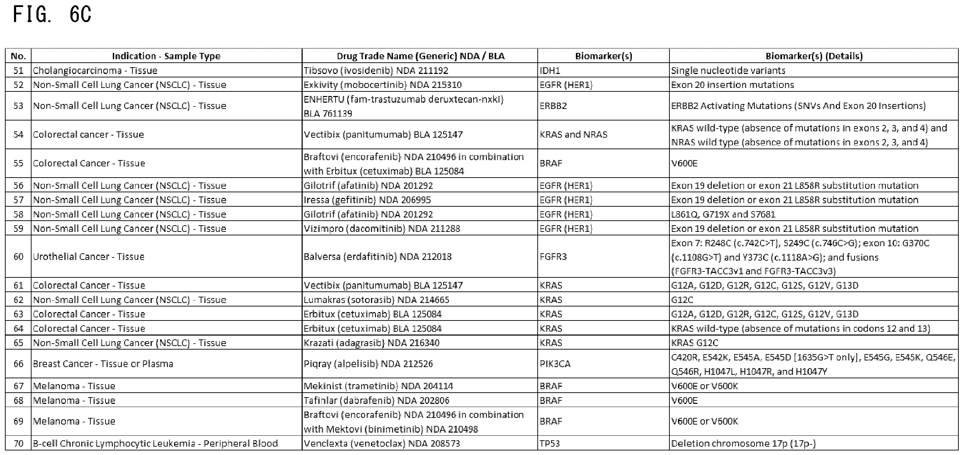

The mutation related to diagnosis or therapy of a disease may be selected from CDx markers approved by the FDA, for example. Specifically, the mutation related to diagnosis or therapy of a disease may be selected from biomarkers described in the tables shown in FIGS. 6A to 6C. Table 3 shows the manufacturer and the name of a test or kit corresponding to each biomarker shown in these drawings.

[Table 3]

| No. | Diagnostic Name (Manufacturer) |
|---|---|
| 1, 2 | Abbott RealTime IDH1 (Abbott Molecular, Inc.) |
| 3 | Abbott RealTime IDH2 (Abbott Molecular, Inc.) |
| 4 | Agilent Resolution ctDx FIRST assay (Resolution Bioscience, Inc.) |
| 5, 6 | cobas 4800 BRAF V600 Mutation Test (Roche Molecular Systems, Inc.) |
| 7 | cobas EGFR Mutation Test v1 (Roche Molecular Systems, Inc.) |
| 8-17 | cobas EGFR Mutation Test v2 (Roche Molecular Systems, Inc.) |
| 18 | cobas EZH2 Mutation Test (Roche Molecular Systems, Inc.) |
| 19, 20 | cobas KRAS Mutation Test (Roche Molecular Systems, Inc.) |
| 21 - 32 | FoundationOne CDx (Foundation Medicine, Inc.) |
| 33 - 37 | FoundationOne Liquid CDx (Foundation Medicine, Inc.) |
| 38 - 41 | Guardant360 CDx (Guardant Health, Inc.) |
| 42 | KIT D816V Assay (ARUP Laboratories, Inc.) |
| 43, 44 | LeukoStrat CDx FLT3 Mutation Assay (Invivoscribe Technologies, Inc.) |
| 45 - 47 | ONCO/Reveal Dx Lung & Colon Cancer Assay (O/RDx-LCCA) (Pillar Biosciences, Inc.) |
| 48 - 53 | Oncomine Dx Target Test (Life Technologies Corporation) |
| 54 | Praxis Extended RAS Panel (Illumina, Inc.) |
| 55 | therascreen BRAF V600E RGQ PCR Kit (QIAGEN GmbH) |
| 56 - 59 | therascreen EGFR RGQ PCR Kit (Qiagen Manchester, Ltd.) |
| 60 | therascreen FGFR RGQ RT-PCR Kit (QIAGEN Manchester Ltd.) |
| 61 - 65 | therascreen KRAS RGQ PCR Kit (Qiagen Manchester, Ltd.) |
| 66 | therascreen PIK3CA RGQ PCR Kit (QIAGEN GmbH) |

(continued)

| No. | Diagnostic Name (Manufacturer) |
|---|---|
| 67 - 69 | THXID BRAF Kit (bioMérieux Inc.) |
| 70 | Vysis CLL FISH Probe Kit (Abbott Molecular, Inc.) |

The predetermined site may be selected from the base sequence in the analysis target region on the basis of the information in the mutation information database 107 above. Specifically, the controller 101 reads out information of the analysis target region and information regarding known gene mutations from the storage unit 105. The information regarding known gene mutations is included in the mutation information database 107. The information regarding known gene mutations is information for specifying the known gene mutation above, the disease information, the drug information, and the biomarker attribution information, for example. On the basis of the read-out information, the controller 101 determines a predetermined site, and extracts information regarding the predetermined site. The information regarding the predetermined site may be stored in the storage unit 105 as information included in the quality index table 109 (see FIG. 2B). The predetermined site may be selected by the controller 101 according to a predetermined determination rule or an instruction from a user, or may be selected by a designer of the computer program for analyzing sequences of nucleic acid stored in the storage unit 105, and the selected predetermined site may be described in advance in the computer program.

As one embodiment, an example of determining the predetermined site on the basis of the biomarker attribution information will be described. In this example, as the biomarker attribution information, the predetermined site is determined on the basis of CDx marker information. However, the biomarker attribution information is not limited to the CDx marker information. The predetermined site may be determined on the basis of information of another biomarker regarding malignancy, prognosis, morbidity risk, onset risk, or the like. The controller 101 obtains CDx marker information from the mutation information database 107. Next, on the basis of the obtained information, the controller 101 extracts the site of the CDx marker from the analysis target region in the analysis target table 108. Then, the controller 101 stores the information indicating the position of the extracted site of the CDx marker, into the quality index table 109 in the storage unit 105. Accordingly, the controller 101 can determine, as the predetermined site, the site of the CDx marker in the analysis target region. Table 4 shows an example of the quality index table 109 of this embodiment. In the table, "Region" indicates the country and region where the CDx marker has been approved.

[Table 4]

| Chr | Gene | AA | Pos | Marker type | Region | Cancer type |
|---|---|---|---|---|---|---|
| 1 | gene A | A146V | 115,252,203 | CDx | JP, US, EP | Colon cancer |
| 2 | gene B | R1275Q | 29,432,664 | CDx | JP, CN | Lung cancer |
| 2 | gene B | G1269A | 29,432,682 | CDx | US | Lung cancer |
| ⋮ | ⋮ | ⋮ | ⋮ | ⋮ | ⋮ | ⋮ |

As shown in Table 4, in the quality index table 109, as the information regarding the predetermined site, the name (Gene) of the gene including the predetermined site, and position information (the chromosome number (Chr) where the predetermined site is positioned, the base number (Pos) in the genome sequence) of the predetermined site are described. As shown in Table 4, in the quality index table 109, as information regarding a mutation having a possibility of occurring at the predetermined site, amino acid change (AA) in the gene mutation is described. In the quality index table 109 shown in Table 4, the type of the marker (Marker type), the country and region (Region) where the CDx test has been approved, and the information (Cancer type) of the type of the disease (cancer) to which the CDx test is applied are further described.

As another embodiment, an example of determining the predetermined site on the basis of the disease information will be described. In this example, the predetermined site is determined on the basis of the name of a disease related to a known gene mutation, as the disease information. More specifically, the predetermined site is determined on the basis of lung cancer. However, the disease information is not limited to lung cancer. The predetermined site may be determined on the basis of information of another disease or disease type. The controller 101 obtains, from the mutation information database 107, information about gene mutations related to lung cancer. Next, on the basis of the obtained information, the controller 101 extracts one base or a plurality of bases related to lung cancer from the analysis target region in the analysis target table 108. Then, the controller 101 stores, as the quality index table 109, the information of the extracted one base or plurality of bases related to lung cancer, into the storage unit 105. Accordingly, the controller 101 can determine, as the predetermined site, the one base or the plurality of bases related to lung cancer in the analysis target region.

An example of determining the predetermined site on the basis of another type of the disease information will be described. In this example, the predetermined site is determined on the basis of information regarding the relevance level between the mutation and the disease, as the disease information. More specifically, the predetermined site is determined on the basis of the evidence level. However, the disease information is not limited to the evidence level. The predetermined site may be determined on the basis of information of the type of the relevance between the mutation and the disease. The controller 101 obtains, from the mutation information database 107, information about gene mutations of which the evidence level is at a predetermined level or higher. Next, on the basis of the obtained information, the controller 101 extracts, from the analysis target region in the analysis target table 108, one base or a plurality of bases where a gene mutation of which the evidence level is at a predetermined level or higher can occur. Then, the controller 101 stores, as the quality index table 109, information of the extracted one base or plurality of bases, into the storage unit 105. Accordingly, the controller 101 can determine, as the predetermined site, the one base or the plurality of bases, in the analysis target region, where a gene mutation of which the evidence level is at a predetermined level or higher can occur.

Further, an example of determining the predetermined site on the basis of another type of the disease information will be described. In this example, the predetermined site is determined on the basis of information regarding a driver mutation, as the disease information. However, the disease information is not limited to the information regarding a driver mutation. The predetermined site may be determined on the basis of information regarding pathogenicity evaluation of the mutation. The controller 101 obtains, from the mutation information database 107, information regarding a driver mutation. Next, on the basis of the obtained information, the controller 101 extracts, from the analysis target region in the analysis target table 108, one base or a plurality of bases where a driver mutation can occur. Then, the controller 101 stores, as the quality index table 109, information of the extracted region, into the storage unit 105. Accordingly, the controller 101 can determine, as the predetermined site, the one base or the plurality of bases, in the analysis target region, where a driver mutation can occur.

Table 5 shows an example of the quality index table 109 based on the disease information. In the table, "Disease type" indicates the type of the disease related to the mutation, "Disease name" indicates the name of the disease related to the mutation, "Evidence type" indicates information of the type of the relevance between the mutation and the disease, and "Evidence level" is information indicating the relevance level between the mutation and the disease. As shown in Table 5, in the quality index table 109, position information (the chromosome number (Chr) where the predetermined site is positioned, the base number (Pos) in the genome sequence) of the predetermined site may be described in association with various types of the disease information.

[Table 5]

| Chr | Gene | AA | Pos | Disease type | Disease name | Evidence type | Evidence level |
|---|---|---|---|---|---|---|---|
| 2 | gene B | R1275Q | 29,432,664 | Cancer | Lung cancer | Predictive | Level A |
| 2 | gene B | G1269A | 29,432,682 | Cancer | Lung cancer | Predictive | Level B |
| ⋮ | ⋮ | ⋮ | ⋮ | ⋮ | ⋮ | ⋮ | ⋮ |

As another embodiment, an example of determining the predetermined site on the basis of the drug information will be described. In this example, the predetermined site is determined on the basis of the presence or absence of a therapeutic drug related to a known gene mutation, as the drug information. However, the drug information is not limited to the presence or absence of a therapeutic drug. The predetermined site may be determined on the basis of information such as the name of a therapeutic drug and the country/region where the therapeutic drug has been approved. The controller 101 obtains, from the mutation information database 107, information about the presence or absence of a therapeutic drug related to a known gene mutation. Next, on the basis of the obtained information, the controller 101 extracts, from the analysis target region in the analysis target table 108, one base or a plurality of bases where a gene mutation of which the relevance to a therapeutic drug is known can occur. Then, the controller 101 stores, as the quality index table 109, information of the one base or the plurality of bases where a gene mutation of which the relevance to a therapeutic drug is known can occur, into the storage unit 105. Accordingly, the controller 101 can determine, as the predetermined site, the one base or the plurality of bases, in the analysis target region, where a gene mutation of which the relevance to a therapeutic drug is known can occur. Table 6 shows an example of the quality index table 109 of this embodiment. In the table, "Drug" indicates an approved therapeutic drug, "Region" indicates a country and a region where the therapeutic drug has been approved, and "Disease name" indicates the cancer type targeted by the approved therapeutic drug. In Table 6, lung cancer is shown as an example of "Disease name".

[Table 6]

| Chr | Gene | AA | Pos | Drug | Region | Disease name |
|---|---|---|---|---|---|---|
| 2 | gene B | R1275Q | 29,432,664 | drug X | JP, CN | Lung cancer |
| 2 | gene B | G1269A | 29,432,682 | drug Y | US | Lung cancer |
| ⋮ | ⋮ | ⋮ | ⋮ | ⋮ | ⋮ | ⋮ |

As shown in Table 6, in the quality index table 109, position information (the chromosome number (Chr) where the predetermined site is positioned, the base number (Pos) in the genome sequence) of the predetermined site may be described in association with various types of the drug information. In the above embodiments, as the position information of the predetermined site, the chromosome number (Chr) where the predetermined site is positioned and the base number (Pos) in the genome sequence are used. However, not limited thereto, any information that specifies the position of the base in the analysis target region may be used.

Regarding step S23 in FIG. 4, on the basis of the second reads, out of the first reads, that correspond to the predetermined site, the controller 101 of the information processing apparatus 10 of the present embodiment generates information regarding the quality of the nucleic acid analysis with respect to the predetermined site. With respect to the nucleic acid derived from the subject, irrespective of the presence or absence of a mutation at the predetermined site, the controller 101 generates information regarding the quality of the nucleic acid analysis with respect to the predetermined site. Here, the "second reads corresponding to the predetermined site" denotes the first reads aligned to a region including the predetermined site in the analysis target region. Hereinafter, the second reads corresponding to the predetermined site will also be simply referred to as "second reads". With reference to FIGS. 7A and 7B, the relationship between the first reads aligned to the analysis target region and the second reads corresponding to the predetermined site will be described. In FIG. 7A, as indicated by "X" and "Y" in the reference sequence, two predetermined sites each composed of one base are selected from the base sequence in the analysis target region. In the drawing, the first reads are indicated by white bars and black bars, and are aligned to the analysis target region. As shown in FIG. 7A, when the predetermined site is composed of one base, the second reads corresponding to the predetermined site are the first reads each including a base at the same position as the predetermined site in the analysis target region. In the drawing, the second reads are indicated by black bars.

As the information regarding the quality of the nucleic acid analysis, depth information at the predetermined site in the analysis target region can be generated, for example. "Depth" denotes the number of reads aligned to the position corresponding to a base on the reference sequence. The depth is also referred to as read depth, and indicates how many times a certain base in the analysis target region has been read in one measurement. "Depth information" denotes depth with respect to the base at the predetermined site and/or information obtained from the depth. For example, when the predetermined site is composed of one base at one place or a plurality of places, the depth information can be the depth with respect to each base. That is, the depth at the predetermined site is the number of the second reads aligned to each base at the predetermined site. When the predetermined site is a region composed of a plurality of bases, the depth information is the depth of each individual base included in the predetermined site and/or a statistically representative value of those depths. Examples of the statistically representative value of the depths include the average value, the mode, the median, the minimum value, the maximum value, or the like of the depths. The depth information is one type of the information regarding the quality of the nucleic acid analysis. In FIG. 7A, with respect to the bases of X and Y at the predetermined site, depth A and depth B are respectively obtained as the depth information.

In FIG. 7B, as the predetermined site, a region from "X" to "Y" in the reference sequence is selected. As in FIG. 7A, the first reads are indicated by white bars and black bars, and are aligned to the analysis target region. The second reads are indicated by black bars. As shown in FIG. 7B, when the predetermined site is composed of a plurality of consecutive bases, the second reads corresponding to the predetermined site are the first reads including bases corresponding to a part or all of the predetermined site. In FIG. 7B, with respect to the predetermined site (the base sequence from X to Y), depth C can be obtained as the depth information.

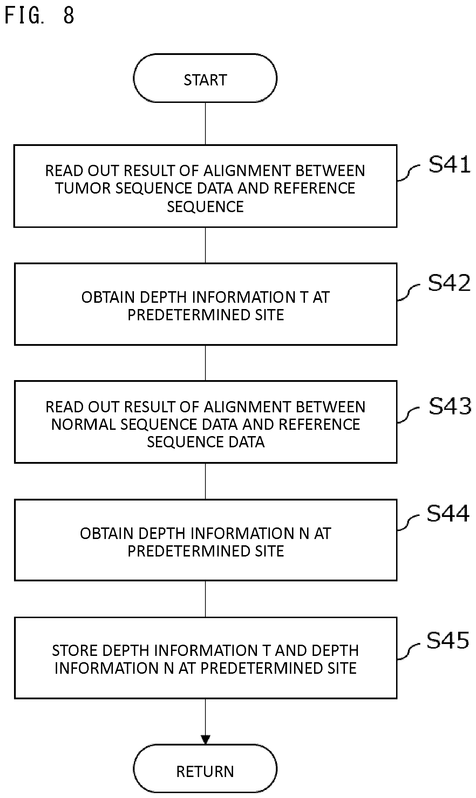

As one embodiment of step S23, a flow of a process of obtaining the depth information at the predetermined site on the basis of each of tumor sequence data and normal sequence data will be described. Hereinafter, the depth information obtained on the basis of the tumor sequence data will be referred to as "depth information T". In addition, the depth information obtained on the basis of the normal sequence data will be referred to as "depth information N". With reference to FIG. 8, in step S41, the controller 101 reads out, from the storage unit 105, the result of the alignment between the tumor sequence data and the reference sequence executed in step S31. In step S42, the controller 101 refers to the result of the alignment between the tumor sequence data and the reference sequence, and obtains the depth information T on the basis of the number of the second reads, which are the first reads corresponding to the predetermined site in the analysis target region. In step S43, the controller 101 reads out, from the storage unit 105, the result of the alignment between the normal sequence data and the reference sequence executed in step S32. In step S44, the controller 101 refers to the result of the alignment between the normal sequence data and the reference sequence, and obtains the depth information N on the basis of the number of the second reads, which are the first reads corresponding to the predetermined site in the analysis target region. When there are a plurality of predetermined sites, the depth information T and N are obtained with respect to each predetermined site. In step S45, the controller 101 stores the depth information T and N at the predetermined site, into the quality information table in the analysis result database 110 in the storage unit 105. Then, the controller 101 ends the process of generating the information regarding the quality of the nucleic acid analysis with respect to the predetermined site, and the process advances to step S24 in FIG. 4.

In obtainment of the depth information T in step S42, mapping data can be used in referring to the result of the alignment between the tumor sequence data and the reference sequence. Similarly, in obtainment of the depth information N in step S44, mapping data can be used also in referring to the result of the alignment between the normal sequence data and the reference sequence. The mapping data is information of reads including the result of alignment of the reads to the reference sequence. When not referring to the results of the alignments between the tumor sequence data and the reference sequence and between the normal sequence data and the reference sequence in steps S42 and S44, the controller 101 may read out the tumor sequence data and the reference sequence in step S41 and execute alignment between the tumor sequence data and the reference sequence in step S42. Further, the controller 101 may read out the normal sequence data and the reference sequence in step S43 and execute alignment between the normal sequence data and the reference sequence in step S44.

Table 7 shows an example of the quality information table, but the quality information table is not limited thereto. In the table, each piece of information corresponding to "Chr", "Gene", "Pos", and "AA" is information transcribed from the quality index table 109, and is the information regarding the predetermined site. "Depth T" is the depth information T, and "Depth N" is the depth information N. In the example of Table 7, in each of the depth information T and the depth information N, an average value of the depths at the predetermined sites at three places is described. However, the value described therein is not limited thereto. For example, the depth information T and the depth information N may each be the maximum value, a middle value, or the minimum value of a plurality of predetermined sites. The depth information T and the depth information N may each be the depth information T and the depth information N at each predetermined site. "Result of mutation detection" is information indicating whether or not, with respect to each predetermined site, a germ cell mutation or a somatic cell mutation has been determined in the process of step S33 or S35. For example, at the first predetermined site in Table 7, it is indicated that a mutation is present, and at the second and third predetermined sites, it is indicated that no mutation is present. "Result of mutation detection" can be determined based on whether or not a mutation at the predetermined site included in the quality information table is included in the measurement result table. As shown in Table 7, irrespective of whether the result of mutation detection at the predetermined site is YES or NO, that is, irrespective of the presence or absence of a mutation at the predetermined site, the depth information T and the depth information N are included in the quality information table. The depth information T and the depth information N are examples of the information regarding the quality of the nucleic acid analysis with respect to the predetermined site. "Result of mutation detection" may be omitted from the quality information table.

[Table 7]

| Chr | Gene | Pos | AA | Depth T | Depth N | Result of mutation detection |
|---|---|---|---|---|---|---|
| 1 | gene A | 115,252,203 | A146V | 500 | 250 | YES |
| 2 | gene B | 29,432,664 | R1275Q | 480 | 200 | NO |
| 2 | gene B | 29,432,682 | G1269A | 450 | 210 | NO |
| 7 | gene C | ∎ ∎ ∎ | ∎ ∎ ∎ | ∎ ∎ ∎ | ∎ ∎ ∎ | ∎ ∎ ∎ |
| ••• | ••• | ••• | ••• | ••• | ••• | ••• |

Examples of the information regarding the quality of the nucleic acid analysis with respect to the predetermined site include information described in (1) to (4) below.

(1) Information regarding the number and/or length of the second reads,
(2) information, provided to each base of each second read, regarding the accuracy of reading of the base sequence by the sequencer,
(3) information provided to each base of each second read in alignment of mapping the first reads to the reference sequence, the information being regarding the accuracy of the mapping, and
(4) a result of determination of the quality based on the information described in at least one of (1) to (3) above.

Out of the information described in (1), information regarding the number of the second reads is the depth information at the predetermined site, for example. The depth information has been described above. The information regarding the length of the second reads is the average value, the mode, the median, the minimum value, the maximum value, or the like of the lengths of the second reads, for example.

The information described in (2) is an index value, provided to each base of the second read, indicating the correctness of base call, for example. Examples of such an index value include the quality score described above. The quality score provided to each base of the second read may be used, as is, as the information described in (2). Alternatively, a statistically representative value (the average value, the mode, the median, the minimum value, the maximum value, or the like of the quality scores) of the quality scores provided to the respective bases of the second reads may be used as the information described in (2).

The information described in (3) is an index value, provided to each second read, indicating the correctness of the mapping, for example. Examples of such an index value include the mapping quality score described above. The information described in (1) to (3) indicates the quality of the nucleic acid analysis with respect to a short region corresponding to the predetermined site composed of one base or the one base and the vicinity thereof. Therefore, for example, the depth information described in (1) indicates the quality of the nucleic acid analysis at the predetermined site at a higher accuracy than the statistically representative value of the depth with respect to the entire length of one gene.

The determination described in (4) may be performed by comparing a value selected from the information described in (1) to (3) above with a threshold, for example. For example, when the depth at the predetermined site is equal to or greater than a corresponding threshold, it may be determined that the quality of the nucleic acid analysis is good. When the depth at the predetermined site is less than a corresponding threshold, it may be determined that the quality of the nucleic acid analysis is not good. The determination result may be displayed in symbols, digits, characters, or the like. For example, when it has been determined that the quality of the nucleic acid analysis is good, a check mark is displayed in the column of the determination result, and when it has been determined that the quality of the nucleic acid analysis is not good, the column of the determination result may be set to be blank.

With reference to FIG. 4, in step S24, the controller 101 outputs, to the output unit 104, a test result report including the result of mutation detection obtained in step S22 and the information regarding the quality of the nucleic acid analysis with respect to the predetermined site obtained in step S23. The controller 101 may output the test result report to the data transmission and reception apparatus 40 via the network 400.

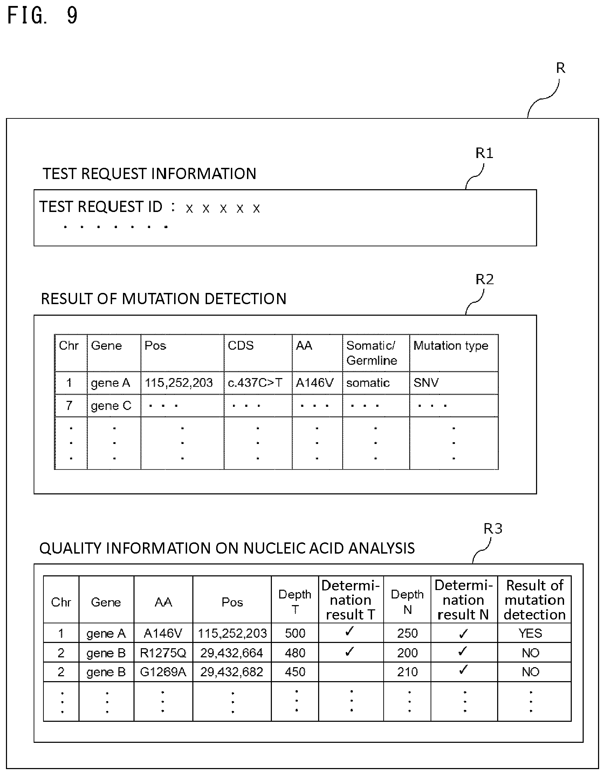

FIG. 9 shows an example of the test result report. A test result report R includes a region R1 in which test request information is described, a region R2 in which the result of mutation detection is described, and a region R3 in which the information regarding the quality of the nucleic acid analysis with respect to the predetermined site is described. In the region R1, the request information transmitted from the request source facility is described, and for example, the test request ID, information of the subject, and the like are described. In the region R2, the result of mutation detection obtained in step S22 and stored in the measurement result table of the storage unit 105 is described. In the region R3, the information regarding the quality of the nucleic acid analysis with respect to the predetermined site obtained in step S23 and stored in the quality information table of the storage unit 105 is described. In the example in FIG. 9, in the region R3, the result of determination of the quality of the nucleic acid analysis is further described. In the table in FIG. 9, "Determination result T" indicates the result of determination of the quality of the nucleic acid analysis, based on the value of the Depth T and a threshold, and "Determination result N" indicates the result of determination of the quality of the nucleic acid analysis, based on the value of the Depth N and a threshold.

As shown in FIG. 9, in the region R3 of the test result report outputted from the information processing apparatus 10, irrespective of the presence or absence of a mutation at the predetermined site, information regarding the quality of the nucleic acid analysis is described. Therefore, even when no mutation has been detected at the predetermined site in the analysis target region of the nucleic acid of the subject, the person in charge in the request source facility 200 and/or the request destination facility 300 can confirm the quality of the nucleic acid analysis with respect to the predetermined site. For example, with respect to the second predetermined site (position 29,432,664 in chromosome 2) in the region R3, the result of mutation detection is "NO" and a check mark is displayed in the column of the determination result T of the Depth T. This suggests that, in this test, no mutation has been detected at the predetermined site and that the result is based on analysis of a sufficient quality. That is, it is suggested that the result of mutation detection is true negative. With respect to the third predetermined site (position 29,432,682 in chromosome 2) in the region R3, the result of mutation detection is "NO" and the column of the determination result T of the Depth T is blank. This suggests that, in this test, although no mutation has been detected at the predetermined site, there is a suspicion, in actuality, that a mutation is present in the nucleic acid of this subject. That is, it is suspected that the result of mutation detection is false negative. In this case, it becomes possible to specifically consider the next action that the medical worker should take, such as performing gene panel testing again, or performing another test focused on a gene marker corresponding to this predetermined site. When a mutation is detected in a re-test, this can lead to an appropriate diagnosis or therapy.

In the present embodiment, the result of mutation detection and the information regarding the quality of the nucleic acid analysis with respect to the predetermined site are described in the same report. However, the result of mutation detection and the information regarding the quality of the nucleic acid analysis with respect to the predetermined site may be generated as separate reports. For example, an analysis result report for reporting the result of mutation detection and a quality control report for reporting the information regarding the quality of the nucleic acid analysis with respect to the predetermined site may be separately generated.

The test result report may further include another type of quality information. The other type of quality information is quality information provided in tests in conventional genomic medicine, and examples thereof include quality information obtained on the basis of the nucleic acid sequence data of the entire analysis target region.

Examples of the other type of quality information include the total number of obtained reads, the mapping ratio, the overlapping ratio, homogeneity, a statistically representative value of the depth in the analysis target region, the frequency distribution of depth frequency in the analysis target region, the frequency distribution of the average quality score of the reads, the frequency distribution of the lengths of the reads, and the like. "Mapping ratio" denotes the proportion of the number of reads, out of the total reads, that have been mapped to the analysis target region. "Overlapping ratio" denotes the proportion of the number of reads, out of the reads mapped to the analysis target region, that are derived from the same amplification region. "Homogeneity" denotes the proportion of the region, out of the analysis target region, where the number of reads having been mapped is equal to or greater than a predetermined value. "Frequency distribution of depth frequency" denotes a histogram that is obtained by summing the number of reads mapped to each base in the analysis target region, and of which the two axes represent the position of the base and the depth with respect to the base. "Frequency distribution of the average quality score of the reads" denotes a histogram of which the two axes represent the average value of the quality scores of the reads and the number of reads having the average value. "Frequency distribution of the lengths of the reads" denotes a histogram of which the two axes represent the length (the number of bases) of a read and the number of reads having the length.

When the mutations detected in step S22 include a mutation detected at a site not described in the quality information table, the test result report may further include information (e.g., the depth information) regarding the quality of the nucleic acid analysis with respect to the site.

With reference to FIG. 4, in step S25, the controller 101 stores the test result report into the report table in the storage unit 105.

(Determination of predetermined site by user)

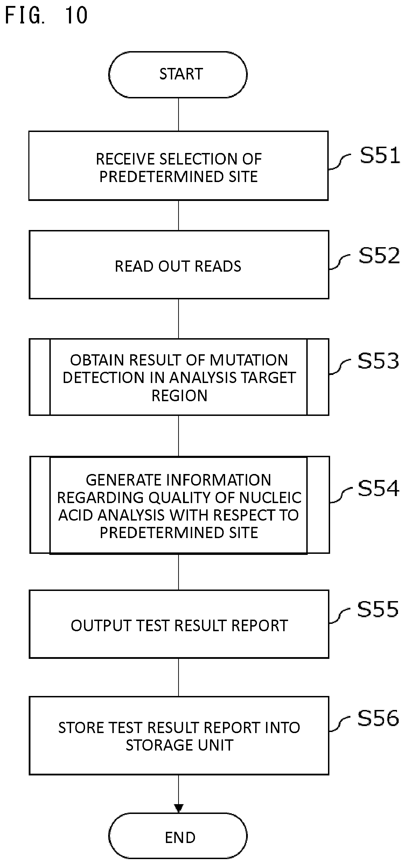

In another embodiment, the user can determine the predetermined site. The user may be a user of the information processing apparatus 10 in the request destination facility 300 to which a test is requested. The user of the information processing apparatus 10 can be a person in charge of tests in the request destination facility 300, for example. In this embodiment, the predetermined site is determined on the basis of information inputted by the user. In the following, an example in which the predetermined site is determined by the user of the information processing apparatus 10 and reads are analyzed will be described with reference to FIG. 10. In the present embodiment, a quality index table obtained by integrating, for example, the quality index tables shown in Tables 4 to 6 is stored as the quality index table 109 of the storage unit 105. In step S51, the controller 101 selects a predetermined site with reference to the quality index table 109, on the basis of the information inputted by the user operating the input unit 103. Input of the information by the user may be performed through a screen displayed on the output unit 104 such as a display or a touch panel. Details of steps S52 to S56 are the same as those described with respect to steps S21 to S25 in FIG. 4. The user is not limited in particular. For example, the user may be a user of the data transmission and reception apparatus 40 in the request source facility 200 which requests the test. The user of the data transmission and reception apparatus 40 can be a medical worker such as a doctor in charge of the subject, for example. The inputting operation performed for determination of the predetermined site is the same between the users of the respective facilities. When the user is the user of the data transmission and reception apparatus 40, the controller 101 receives, in step S51 via the network 400, the information inputted by the user through a screen displayed on the data transmission and reception apparatus 40.

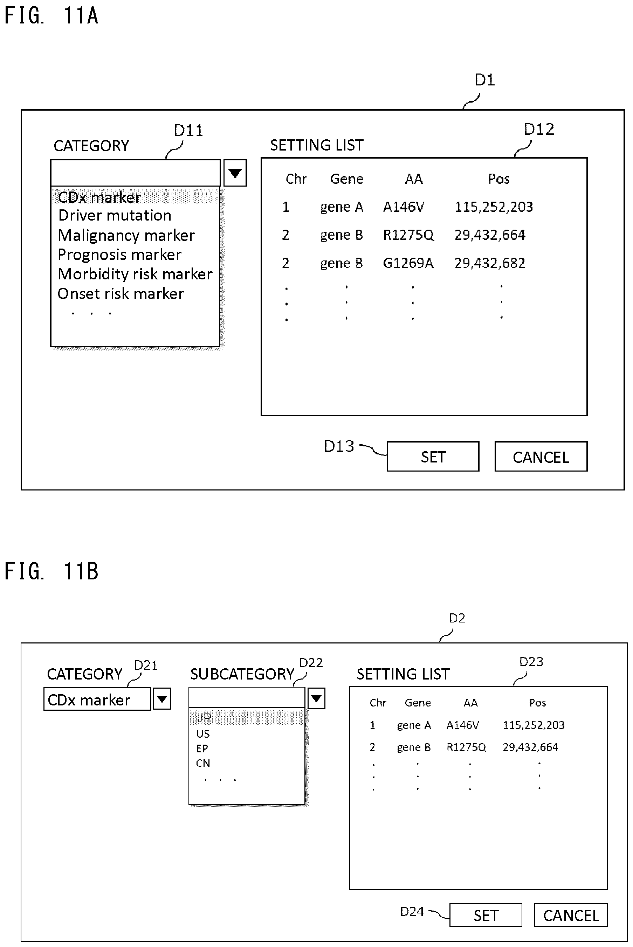

With reference to FIG. 11A, an example of selecting the predetermined site by inputting category information on a screen D1 will be described. In D11 on the screen D1, as category information, the biomarker attribution information is displayed in a selectable manner. Inputting of the category information is performed by selecting biomarker attribution information from a pulldown menu, for example. When category information has been inputted in D11, the controller 101 reads out, from the storage unit 105, information regarding the predetermined site corresponding to the category information. Then, information regarding the read out predetermined site is displayed in D12. When the user selects one or a plurality of predetermined sites displayed in D12 by clicking or the like, and then clicks a setting button in D13, information of the selected predetermined site is stored into the storage unit 105. When the user is the user of the data transmission and reception apparatus 40 in the request source facility 200, information of the selected predetermined site is transmitted to the information processing apparatus 10 via the network 400, together with the request information.

With reference to FIG. 11B, an example of selecting the predetermined site by inputting category information and subcategory information on a screen D2 will be described. In D21 on the screen D2, the CDx marker has been selected as the category information. In this case, in D22, as the subcategory information, for example, information of the country/region where the CDx marker has been approved is displayed in a selectable manner. When the category information and the subcategory information have been inputted, the controller 101 reads out, from the storage unit 105, information regarding the predetermined site corresponding to those pieces of information, and the read out information regarding the predetermined site is displayed in D23. When the user selects one or a plurality of predetermined sites displayed in D23 by clicking or the like, and then clicks a setting button in D24, information of the selected predetermined site is stored into the storage unit 105. Accordingly, from the analysis target region, the CDx marker approved in a certain country/region can be set as the predetermined site. The subcategory information may be, other than the country/region, a disease name, a therapeutic drug, or the like to which the CDx test is applied.

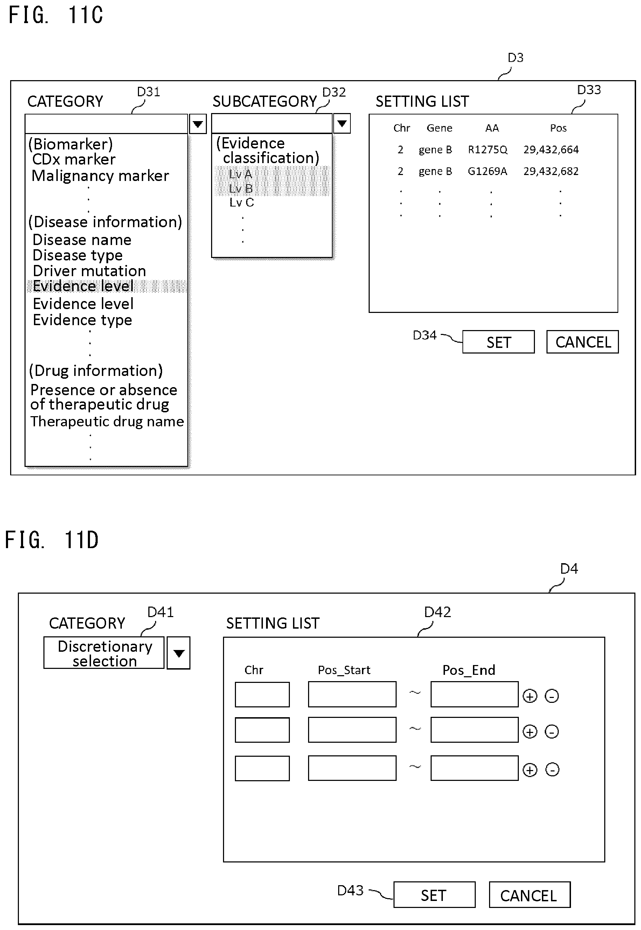

The category information may be other than the biomarker attribution information. With reference to FIG. 11C, in D31 on a screen D3, as the category information, not only the biomarker attribution information but also the disease information, the drug information, and the like are displayed in a selectable manner. In D31 on the screen D3, the evidence level is selected as the category information. In this case, in D32, as the subcategory information, classification (e.g., Lv A, Lv B, Lv C, etc.) of the evidence level is displayed in a selectable manner. When the category information and the subcategory information have been inputted, the controller 101 reads out, from the storage unit 105, information regarding the predetermined site corresponding to those pieces of information, and the read out information regarding the predetermined site is displayed in D33. When the user selects one or a plurality of predetermined sites displayed in D33 by clicking or the like, and then clicks a setting button in D34, information of the selected predetermined site is stored into the storage unit 105.

The predetermined site may be determined by the user inputting a discretionary region from the analysis target region. With reference to FIG. 11D, in D41 on a screen D4, "discretionary selection" is selected as the category information. In this case, in D42, text boxes for inputting position information such as the chromosome number (Chr) and the base number (Pos_Start and Pos End) on the genome sequence are displayed. The user inputs, into the text boxes, position information of desired one base or a plurality of bases as the predetermined site, out of the base sequence in the analysis target region. When the user has inputted the position information and then clicks a setting button in D43, the inputted information of the predetermined site is stored into the storage unit 105.

(Detection of somatic cell mutation without matched pair test)

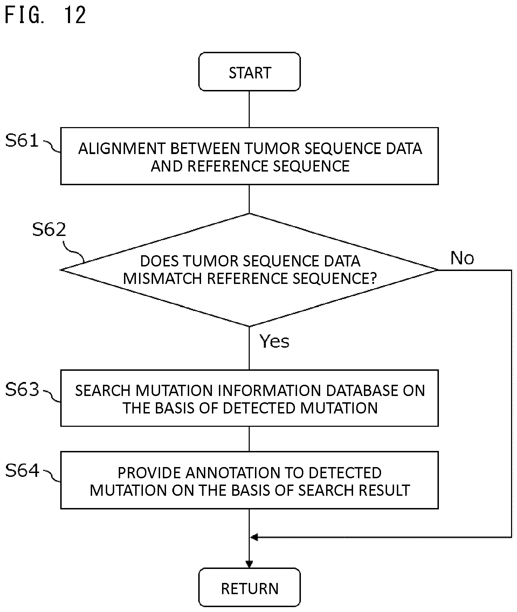

As another embodiment of step S22 in FIG. 4, a flow of a process of detecting a mutation without the matched pair test will be described. In this case, the non-tumor specimen is not used, and reads obtained from a library sample prepared from a tumor specimen are used as the tumor sequence data. With reference to FIG. 12, in step S61, the controller 101 reads out a reference sequence from the reference sequence database 106 and executes alignment between the tumor sequence data and the reference sequence. In step S62, the controller 101 determines whether or not there is a site, in the tumor sequence data, that does not match the reference sequence. When the controller 101 has determined that the tumor sequence data does not match the reference sequence, the process advances to step S63. In the process in FIG. 12, when there is a site that does not match the reference sequence, it is determined that the site is that of a mutation observed in a gene of the tumor tissue. In step S62, when the tumor sequence data matches the reference sequence, the controller 101 ends the process. In this case, it is considered to that there is no mutation in the tumor sequence data.

In step S63, the controller 101 searches the mutation information database 107 on the basis of the detected mutation. In step S64, the controller 101 provides an annotation to the detected mutation on the basis of the search result. Details of steps S63 and S64 are the same as those described with respect to steps S37 and S38 in FIG. 5. Through the processes of steps S61 to S64, the controller 101 obtains, in step S22, information of the gene mutation in the analysis target region, as the result of mutation detection.

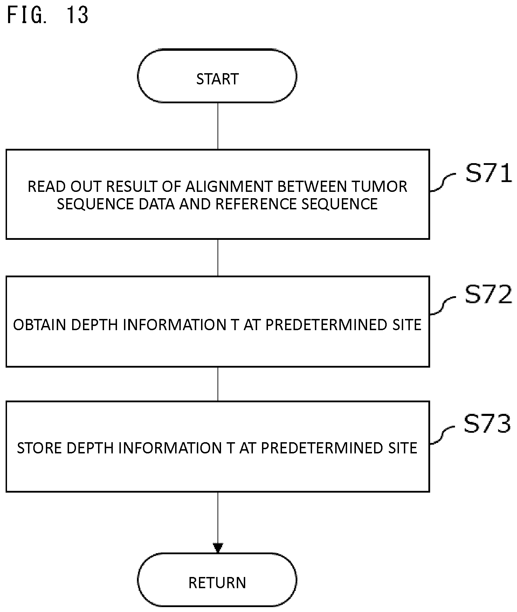

As another embodiment of step S23 in FIG. 4, a flow of a process of obtaining the depth information T at the predetermined site on the basis of the tumor sequence data will be described. With reference to FIG. 13, in step S71, the controller 101 reads out the alignment result between the tumor sequence data and the reference sequence obtained in step S61. In step S72, the controller 101 refers to the result of the alignment between the tumor sequence data and the reference sequence, and obtains the depth information T on the basis of the number of the second reads, which are the first reads corresponding to the predetermined site in the analysis target region. As the depth information T, the depth with respect to each base at the predetermined site is preferable. In step S73, the controller 101 stores the depth information T at the predetermined site into the storage unit 105. Then, the controller 101 ends the process of obtaining the depth information at the predetermined site, and the process advances to step S24 in FIG. 4.

In addition to the above, various modifications of the embodiments of the present invention may be made as appropriate without departing from the scope of the technical idea defined by the claims.

[Remarks]

The present disclosure includes the following items 1-25.
Item 1: A method for controlling an information processing apparatus configured to analyze a sequence of nucleic acid obtained from a specimen of a subject, the method comprising:

obtaining, on the basis of a plurality of first reads read by a sequencer, a result of mutation detection in an analysis target region in the sequence of the nucleic acid;
generating, on the basis of second reads, out of the first reads, that correspond to at least one predetermined site selected from a base sequence in the analysis target region, information regarding a quality of a nucleic acid analysis with respect to the predetermined site; and
outputting the information regarding the quality, irrespective of presence or absence of a mutation at the predetermined site.

Item 2: The method of item 1, further comprising
outputting the result of mutation detection in the analysis target region.
Item 3: The method of item 1, further comprising
receiving a selection of the predetermined site from the analysis target region.
Item 4: The method of item 3, wherein

the receiving of the selection comprises receiving an input of information regarding the predetermined site, and
the predetermined site is set on the basis of the information having been inputted.

Item 5: The method of item 1, wherein
the information regarding the quality is generated with respect to each of a plurality of the predetermined sites selected from the base sequence in the analysis target region.
Item 6: The method of item 1, wherein
the predetermined site is composed of one base or two or more consecutive bases in the base sequence in the analysis target region.
Item 7: The method of item 1, wherein
the predetermined site includes a site having a possibility of occurrence of a mutation.
Item 8: The method of item 7, wherein
the mutation having the possibility of occurring at the predetermined site is a mutation, in the analysis target region, related to diagnosis or therapy of a disease.
Item 9: The method of item 8, wherein
the mutation related to diagnosis or therapy of a disease is at least one of

(1) a mutation known to be related to an effect and/or a side effect of a drug,
(2) a mutation known to be related to a state, onset, and/or prognosis of a disease, and
(3) a mutation of which an evidence level indicating relevance to diagnosis or therapy is known to be at a predetermined level or higher.

Item 10:The method of item 9, wherein
the mutation described in said (1) is a companion diagnostics marker.
Item 11 :The method of item 9, wherein
the mutation described in said (2) is at least one selected from the group consisting of a driver mutation, a pathogenic mutation, a mutation as a malignancy marker, a mutation as a prognostic marker, a mutation as a morbidity risk marker, or a mutation as an onset risk marker.
Item 12:The method of item 9, wherein
the evidence level described in said (3) is an evidence level of at least one selected from the group consisting of oncogenic evidence, predisposing evidence, predictive evidence, diagnostic evidence, or prognostic evidence.
Item 13:The method of item 8, wherein
the mutation related to diagnosis or therapy of a disease is at least one selected from biomarkers described in tables shown in FIG. 6A to FIG. 6C.
Item 14:The method of item 1, wherein
the predetermined site is a site selected from the base sequence in the analysis target region on the basis of information of a mutation information database in which information of known gene mutations is accumulated.

Item 15:The method of item 1, wherein

the information regarding the quality is at least one selected from the group consisting of

(1) information regarding the number and/or a length of the second reads,
(2) information, provided to each base of each second read, regarding accuracy of reading of the base sequence by the sequencer,
(3) information provided to each base of each second read in alignment of mapping the first reads to a reference sequence, the information being regarding accuracy of the mapping, and
(4) a result of determination of a quality based on the information described in at least one of said (1) to (3).

Item 16:The method of item 15, wherein

the information described in said (1) is at least one selected from depth information at the predetermined site and an average value of the lengths of the second reads.

Item 17:The method of item 15, wherein

the information described in said (2) is an index value, provided to each base of the second read, indicating correctness of base call.

Item 18:The method of item 15, wherein

the information described in said (3) is an index value, provided to the second read, indicating correctness of the mapping.

Item 19:The method of item 1, wherein

the information regarding the quality is outputted in association with information regarding the predetermined site.

Item 20:The method of item 1, wherein

the outputting of the information regarding the quality comprises generating and outputting a quality control report including the information regarding the quality of the nucleic acid analysis with respect to the predetermined site.

Item 21 :The method of item 2, wherein

the outputting of the information regarding the quality comprises generating and outputting an analysis result report including the result of mutation detection in the analysis target region.

Item 22:The method of item 1, wherein

the analysis target region is a whole-genome region, a whole-exome region, or a part of a genome region including at least one target gene.

Item 23:The method of item 1, wherein

the nucleic acid obtained from the specimen of the subject is DNA or RNA.

Item 24:An information processing apparatus configured to analyze a sequence of nucleic acid obtained from a specimen of a subject,

the information processing apparatus comprising a controller and an output unit,
the controller being configured to execute:

obtaining, on the basis of a plurality of first reads read by a sequencer, a result of mutation detection in an analysis target region in the sequence of the nucleic acid;
generating, on the basis of second reads, out of the first reads, that correspond to at least one predetermined site selected from a base sequence in the analysis target region, information regarding a quality of a nucleic acid analysis with respect to the predetermined site; and
outputting, to the output unit, the information regarding the quality, irrespective of presence or absence of a mutation at the predetermined site.

Item 25:A computer program for analyzing a sequence of nucleic acid obtained from a specimen of a subject, the computer program being stored in a computer-readable medium and causing the computer to execute:

obtaining, on the basis of a plurality of first reads read by a sequencer, a result of mutation detection in an analysis target region in the sequence of the nucleic acid;
generating, on the basis of second reads, out of the first reads, that correspond to at least one predetermined site selected from a base sequence in the analysis target region, information regarding a quality of a nucleic acid analysis with respect to the predetermined site; and
outputting the information regarding the quality, irrespective of presence or absence of a mutation at the predetermined site.

**Claims**

1.  A method for controlling an information processing apparatus configured to analyze a sequence of nucleic acid obtained from a specimen of a subject, the method comprising:

    obtaining, on the basis of a plurality of first reads read by a sequencer, a result of mutation detection in an analysis target region in the sequence of the nucleic acid;
    generating, on the basis of second reads, out of the first reads, that correspond to at least one predetermined site selected from a base sequence in the analysis target region, information regarding a quality of a nucleic acid analysis with respect to the predetermined site; and
    outputting the information regarding the quality, irrespective of presence or absence of a mutation at the predetermined site.

2.  The method of claim 1, further comprising
    outputting the result of mutation detection in the analysis target region.

3.  The method of claim 1, further comprising
    receiving a selection of the predetermined site from the analysis target region.

4.  The method of claim 3, wherein

    the receiving of the selection comprises receiving an input of information regarding the predetermined site, and
    the predetermined site is set on the basis of the information having been inputted.

5.  The method of claim 1, wherein
    the information regarding the quality is generated with respect to each of a plurality of the predetermined sites selected from the base sequence in the analysis target region.

6.  The method of claim 1, wherein
    the predetermined site is composed of one base or two or more consecutive bases in the base sequence in the analysis target region.

7.  The method of claim 1, wherein
    the predetermined site includes a site having a possibility of occurrence of a mutation.

8.  The method of claim 7, wherein
    the mutation having the possibility of occurring at the predetermined site is a mutation, in the analysis target region, related to diagnosis or therapy of a disease.

9.  The method of claim 8, wherein
    the mutation related to diagnosis or therapy of a disease is at least one of

    (1) a mutation known to be related to an effect and/or a side effect of a drug,
    (2) a mutation known to be related to a state, onset, and/or prognosis of a disease, and
    (3) a mutation of which an evidence level indicating relevance to diagnosis or therapy is known to be at a predetermined level or higher.

10. The method of claim 9, wherein

    the mutation described in said (1) is a companion diagnostics marker,
    the mutation described in said (2) is at least one selected from the group consisting of a driver mutation, a pathogenic mutation, a mutation as a malignancy marker, a mutation as a prognostic marker, a mutation as a morbidity risk marker, or a mutation as an onset risk marker, and

    the evidence level described in said (3) is an evidence level of at least one selected from the group consisting of oncogenic evidence, predisposing evidence, predictive evidence, diagnostic evidence, or prognostic evidence.

11. The method of claim 8, wherein

the mutation related to diagnosis or therapy of a disease is at least one selected from biomarkers described in tables shown in FIG. 6A to FIG. 6C.

12. The method of claim 1, wherein
the predetermined site is a site selected from the base sequence in the analysis target region on the basis of information of a mutation information database in which information of known gene mutations is accumulated.

13. The method of claim 1, wherein
the information regarding the quality is at least one selected from the group consisting of

(1) information regarding the number and/or a length of the second reads,
(2) information, provided to each base of each second read, regarding accuracy of reading of the base sequence by the sequencer,
(3) information provided to each base of each second read in alignment of mapping the first reads to a reference sequence, the information being regarding accuracy of the mapping, and
(4) a result of determination of a quality based on the information described in at least one of said (1) to (3).

14. The method of claim 13, wherein

the information described in said (1) is at least one selected from depth information at the predetermined site and an average value of the lengths of the second reads,
the information described in said (2) is an index value, provided to each base of the second read, indicating correctness of base call, and
the information described in said (3) is an index value, provided to the second read, indicating correctness of the mapping.

15. The method of claim 1, wherein
the information regarding the quality is outputted in association with information regarding the predetermined site.

16. The method of claim 1, wherein
the outputting of the information regarding the quality comprises generating and outputting a quality control report including the information regarding the quality of the nucleic acid analysis with respect to the predetermined site.

17. The method of claim 2, wherein
the outputting of the information regarding the quality comprises generating and outputting an analysis result report including the result of mutation detection in the analysis target region.

18. An information processing apparatus configured to analyze a sequence of nucleic acid obtained from a specimen of a subject,

the information processing apparatus comprising a controller and an output unit,
the controller being configured to execute:

obtaining, on the basis of a plurality of first reads read by a sequencer, a result of mutation detection in an analysis target region in the sequence of the nucleic acid;
generating, on the basis of second reads, out of the first reads, that correspond to at least one predetermined site selected from a base sequence in the analysis target region, information regarding a quality of a nucleic acid analysis with respect to the predetermined site; and
outputting, to the output unit, the information regarding the quality, irrespective of presence or absence of a mutation at the predetermined site.

19. A computer program for analyzing a sequence of nucleic acid obtained from a specimen of a subject,
the computer program being stored in a computer-readable medium and causing the computer to execute:

obtaining, on the basis of a plurality of first reads read by a sequencer, a result of mutation detection in an analysis target region in the sequence of the nucleic acid;
generating, on the basis of second reads, out of the first reads, that correspond to at least one predetermined site selected from a base sequence in the analysis target region, information regarding a quality of a nucleic acid analysis with respect to the predetermined site; and

outputting the information regarding the quality, irrespective of presence or absence of a mutation at the predetermined site.

FIG. 1

FIG. 2A

FIG. 2B

110

TEST REQUEST ID

| MEASUREMENT RESULT TABLE |

| QUALITY INFORMATION TABLE |

| REPORT TABLE |

FIG. 2C

30

20

STORAGE

10

302

303 | INPUT UNIT

304 | OUTPUT UNIT

TRANSMISSION AND RECEPTION UNIT

INFORMATION PROCESSING APPARATUS

306 | FLOW CELL

CONTROLLER

307 | IMAGING UNIT

301

STORAGE UNIT

305

FIG. 3

```
            ┌─────────────────┐
            │      START       │
            └─────────────────┘
                     │
   ┌─────────────────────────────────────┐
   │   RECEIVE REQUEST FOR ANALYSIS       │  ⌐ S11
   └─────────────────────────────────────┘
                     │
   ┌─────────────────────────────────────┐
   │  READ BASE SEQUENCE OF LIBRARY SAMPLE │  ⌐ S12
   └─────────────────────────────────────┘
                     │
   ┌─────────────────────────────────────┐
   │    TRANSMIT READ TO STORAGE 20        │  ⌐ S13
   └─────────────────────────────────────┘
                     │
                     ▼
            ┌─────────────────┐
            │       END        │
            └─────────────────┘
```

FIG. 4

```
                    ┌─────────────┐
                    │    START    │
                    └──────┬──────┘
                           │
         ┌─────────────────────────────────────┐
         │          READ OUT READS             │── S21
         └─────────────────┬───────────────────┘
                           │
         ┌─────────────────────────────────────┐
         │ ║  OBTAIN RESULT OF MUTATION     ║  │── S22
         │ ║  DETECTION IN ANALYSIS TARGET  ║  │
         │ ║           REGION               ║  │
         └─────────────────┬───────────────────┘
                           │
         ┌─────────────────────────────────────┐
         │ ║ GENERATE INFORMATION REGARDING ║  │── S23
         │ ║ QUALITY OF NUCLEIC ACID ANALYSIS║ │
         │ ║ WITH RESPECT TO PREDETERMINED  ║  │
         │ ║            SITE                ║  │
         └─────────────────┬───────────────────┘
                           │
         ┌─────────────────────────────────────┐
         │       OUTPUT TEST RESULT REPORT     │── S24
         └─────────────────┬───────────────────┘
                           │
         ┌─────────────────────────────────────┐
         │ STORE TEST RESULT REPORT INTO STORAGE│── S25
         │               UNIT                  │
         └─────────────────┬───────────────────┘
                           │
                    ┌──────▼──────┐
                    │     END     │
                    └─────────────┘
```

FIG. 5

START

S31 — ALIGNMENT BETWEEN TUMOR SEQUENCE DATA AND REFERENCE SEQUENCE

S32 — ALIGNMENT BETWEEN NORMAL SEQUENCE DATA AND REFERENCE SEQUENCE

S33 — DOES NORMAL SEQUENCE DATA MATCH REFERENCE SEQUENCE?

No

S34 — DETERMINE THAT DETECTED SITE IS THAT OF GERM CELL MUTATION

Yes

S35 — DOES TUMOR SEQUENCE DATA MISMATCH REFERENCE SEQUENCE?

No

Yes

S36 — DETERMINE DETECTED SITE TO BE THAT OF SOMATIC CELL MUTATION

S37 — SEARCH MUTATION INFORMATION DATABASE ON THE BASIS OF DETECTED SOMATIC CELL MUTATION OR GERM CELL MUTATION

S38 — PROVIDE ANNOTATION TO DETECTED SOMATIC CELL MUTATION OR GERM CELL MUTATION ON THE BASIS OF SEARCH RESULT

RETURN

# FIG. 6A

| No. | Indication - Sample Type | Drug Trade Name (Generic) NDA / BLA | Biomarker(s) | Biomarker(s) (Details) |
|---|---|---|---|---|
| 1 | Acute Myeloid Leukemia - Peripheral Blood or Bone Marrow | Tibsovo (ivosidenib) NDA 211192 | IDH1 | R132 mutations (R132C, R132H, R132G, R132S, and R132L) |
| 2 | Acute Myeloid Leukemia - Peripheral Blood or Bone Marrow | Rezlidhia (olutasidenib) NDA 215814 | IDH1 | R132 mutations (R132C, R132H, R132G, R132S, and R132L) |
| 3 | Acute Myeloid Leukemia - Peripheral Blood or Bone Marrow | Idhifa (enasidenib) NDA 209606 | IDH2 | R140Q, R140L, R140G, R140W, R172K, R172M, R172G, R172S, and R172W |
| 4 | Non-Small Cell Lung Cancer (NSCLC) - Plasma | Krazati (adagrasib) NDA 216340 | KRAS | KRAS G12C |
| 5 | Melanoma - Tissue | Zelboraf (vemurafenib) NDA 202429 | BRAF | V600E |
| 6 | Melanoma - Tissue | Cotellic (cobimetinib) NDA 206192 in combination with Zelboraf (vemurafenib) NDA 202429 | BRAF | V600E or V600K |
| 7 | Non-Small Cell Lung Cancer (NSCLC) - Tissue | Tarceva (erlotinib) NDA 021743 | EGFR (HER1) | Exon 19 deletion or exon 21 L858R substitution mutation |
| 8 | Non-Small Cell Lung Cancer (NSCLC) - Tissue | Tagrisso (osimertinib) NDA 208065 | EGFR (HER1) | T790M |
| 9 | Non-Small Cell Lung Cancer (NSCLC) - Tissue | Tagrisso (osimertinib) NDA 208065 | EGFR (HER1) | Exon 19 deletion or exon 21 L858R substitution mutation |
| 10 | Non-Small Cell Lung Cancer (NSCLC) - Plasma | Tagrisso (osimertinib) NDA 208065 | EGFR (HER1) | Exon 19 deletion or exon 21 L858R substitution mutation |
| 11 | Non-Small Cell Lung Cancer (NSCLC) - Tissue or Plasma | Iressa (gefitinib) NDA 206995 | EGFR (HER1) | Exon 19 deletion or exon 21 L858R substitution mutation |
| 12 | Non-Small Cell Lung Cancer (NSCLC) - Tissue or Plasma | Iressa (gefitinib) NDA 206995 | EGFR (HER1) | Exon 19 deletion or exon 21 L858R substitution mutation |
| 13 | Non-Small Cell Lung Cancer (NSCLC) - Tissue or Plasma | Tarceva (erlotinib) NDA 021743 | EGFR (HER1) | Exon 19 deletion or exon 21 L858R substitution mutation |
| 14 | Non-Small Cell Lung Cancer (NSCLC) - Tissue or Plasma | Gilotrif (afatinib) NDA 201292 | EGFR (HER1) | Exon 19 deletion or exon 21 L858R substitution mutation |
| 15 | Non-Small Cell Lung Cancer (NSCLC) - Tissue or Plasma | Tagrisso (osimertinib) NDA 208065 | EGFR (HER1) | Exon 19 deletion or exon 21 L858R substitution mutation |
| 16 | Non-Small Cell Lung Cancer (NSCLC) - Plasma | Tagrisso (osimertinib) NDA 208065 | EGFR (HER1) | T790M |
| 17 | Non-Small Cell Lung Cancer (NSCLC) - Plasma | Tarceva (erlotinib) NDA 021743 | EGFR (HER1) | Exon 19 deletion or exon 21 L858R substitution mutation |
| 18 | Follicular Lymphoma Tumor - Tissue | Tazverik (tazemetostat) NDA 213400 | EZH2 | Y646N, Y646F or Y646X (Y646H, Y646S, or Y646C), A682G, and A692V of the EZH2 gene |
| 19 | Colorectal Cancer - Tissue | Erbitux (cetuximab) BLA 125084 | KRAS | Mutations in codons 12 and 13 of KRAS gene |
| 20 | Colorectal Cancer - Tissue | Vectibix (panitumumab) BLA 125147 | KRAS | Mutations in codons 12 and 13 of KRAS gene |
| 21 | Non-Small Cell Lung Cancer (NSCLC) - Tissue | Gilotrif (afatinib) NDA 201292 | EGFR (HER1) | Exon 19 deletion or exon 21 L858R substitution mutation |
| 22 | Non-Small Cell Lung Cancer (NSCLC) - Tissue | Iressa (gefitinib) NDA 206995 | EGFR (HER1) | Exon 19 deletion or exon 21 L858R substitution mutation |
| 23 | Non-Small Cell Lung Cancer (NSCLC) - Tissue | Tarceva (erlotinib) NDA 021743 | EGFR (HER1) | Exon 19 deletion or exon 21 L858R substitution mutation |
| 24 | Non-Small Cell Lung Cancer (NSCLC) - Tissue | Tagrisso (osimertinib) NDA 208065 | EGFR (HER1) | T790M |
| 25 | Non-Small Cell Lung Cancer (NSCLC) - Tissue | Tafinlar (dabrafenib) NDA 202806 in combination with Mekinist (trametinib) NDA 204114 | BRAF | V600E |
| 26 | Colorectal Cancer - Tissue | Erbitux (cetuximab) BLA 125084 | KRAS | KRAS wild-type (absence of mutations in codons 12 and 13) |
| 27 | Colorectal Cancer - Tissue | Vectibix (panitumumab) BLA 125147 | KRAS and NRAS | KRAS wild-type (absence of mutations in exons 2, 3, and 4) and NRAS wild type (absence of mutations in exons 2, 3, and 4) |

FIG. 6B

| No. | Indication - Sample Type | Drug Trade Name (Generic) NDA / BLA | Biomarker(s) | Biomarker(s) (Details) |
|---|---|---|---|---|
| 28 | Melanoma - Tissue | Mekinist (trametinib) NDA 204114 | BRAF | V600E and V600K |
| 29 | Breast Cancer - Tissue | Piqray (alpelisib) NDA 212526 | PIK3CA | C420R, E542K, E545A, E545D [1635G>T only], E545G, E545K, Q546E, Q546R, H1047L, H1047R, and H1047Y |
| 30 | Non-Small Cell Lung Cancer (NSCLC) - Tissue | Tagrisso (osimertinib) NDA 208065 | EGFR (HER1) | Exon 19 deletion or exon 21 L858R substitution mutation |
| 31 | Non-Small Cell Lung Cancer (NSCLC) - Tissue | Tabrecta (capmatinib) NDA 213591 | MET | MET single nucleotide variants and indels that lead to MET exon 14 skipping |
| 32 | Melanoma - Tissue | Tecentriq (atezolizumab) BLA 761034 in combination with Cotellic (cobimetinib) NDA 206192 and Zelboraf (vemurafenib) NDA 202429 | BRAF | BRAF V600 mutations |
| 33 | Non-Small Cell Lung Cancer (NSCLC) - Plasma | Iressa (gefitinib) NDA 206995 | EGFR (HER1) | Exon 19 deletion or exon 21 L858R substitution mutation |
| 34 | Non-Small Cell Lung Cancer (NSCLC) - Plasma | Tagrisso (osimertinib) NDA 208065 | EGFR (HER1) | Exon 19 deletion or exon 21 L858R substitution mutation |
| 35 | Non-Small Cell Lung Cancer (NSCLC) - Plasma | Tarceva (erlotinib) NDA 021743 | EGFR (HER1) | Exon 19 deletion or exon 21 L858R substitution mutation |
| 36 | Non-Small Cell Lung Cancer (NSCLC) - Plasma | Tabrecta (capmatinib) NDA 213591 | MET | MET single nucleotide variants and indels that lead to MET exon 14 skipping |
| 37 | Breast Cancer - Plasma | Piqray (alpelisib) NDA 212526 | PIK3CA | C420R, E542K, E545A, E545D [1635G>T only], E545G, E545K, Q546E, Q546R, H1047L, H1047R, and H1047Y |
| 38 | Non-Small Cell Lung Cancer (NSCLC) - Plasma | Tagrisso (osimertinib) NDA 208065 | EGFR (HER1) | EGFR exon 19 deletions, EGFR exon 21 L858R, and T790M |
| 39 | Non-Small Cell Lung Cancer (NSCLC) - Plasma | Rybrevant (amivantamb) BLA 761210 | EGFR (HER1) | EGFR exon 20 insertions |
| 40 | Non-Small Cell Lung Cancer (NSCLC) - Plasma | Lumakras (sotorasib) NDA 214665 | KRAS | G12C |
| 41 | Non-Small Cell Lung Cancer (NSCLC) - Plasma | ENHERTU (fam-trastuzumab deruxtecan-nxki) BLA 761139 | ERBB2 | ERBB2 Activating Mutations (SNVs And Exon 20 Insertions) |
| 42 | Aggressive Systemic Mastocytosis - Bone Marrow | Gleevec (imatinib mesylate) NDA 021588 | KIT | D816V |
| 43 | Acute Myelogenous Leukemia - Peripheral Blood or Bone Marrow | Rydapt (midostaurin) NDA 207997 | FLT3 (ITD/TDK) | ITD mutations and TKD mutations D835 and I836 |
| 44 | Acute Myelogenous Leukemia - Peripheral Blood or Bone Marrow | Xospata (gilteritinib) NDA 211349 | FLT3 (ITD/TDK) | ITD mutations and TKD mutations D835 and I836 |
| 45 | Colorectal Cancer - Tissue | Erbitux (cetuximab) BLA 125084 | KRAS | KRAS wild-type (absence of mutations in codons 12 and 13) |
| 46 | Colorectal Cancer - Tissue | Vectibix (panitumumab) BLA 125147 | KRAS | KRAS wild-type (absence of mutations in codons 12 and 13) |
| 47 | Non-Small Cell Lung Cancer (NSCLC) - Tissue | A tyrosine kinase inhibitor approved by FDA for that indication | EGFR (HER1) | Exon 19 deletion or exon 21 L858R substitution mutation |
| 48 | Non-Small Cell Lung Cancer (NSCLC) - Tissue | Tafinlar (dabrafenib) NDA 202806 in combination with Mekinist (trametinib) NDA 204114 | BRAF | V600E |
| 49 | Non-Small Cell Lung Cancer (NSCLC) - Tissue | Iressa (gefitinib) NDA 206995 | EGFR (HER1) | Exon 19 deletion or exon 21 L858R substitution mutation |
| 50 | Non-Small Cell Lung Cancer (NSCLC) - Tissue | Rybrevant (amivantamb) BLA 761210 | EGFR (HER1) | Exon 20 insertion mutations |

# FIG. 6C

| No. | Indication - Sample Type | Drug Trade Name (Generic) NDA / BLA | Biomarker(s) | Biomarker(s) (Details) |
|---|---|---|---|---|
| 51 | Cholangiocarcinoma - Tissue | Tibsovo (ivosidenib) NDA 211192 | IDH1 | Single nucleotide variants |
| 52 | Non-Small Cell Lung Cancer (NSCLC) - Tissue | Exkivity (mobocertinib) NDA 215310 | EGFR (HER1) | Exon 20 insertion mutations |
| 53 | Non-Small Cell Lung Cancer (NSCLC) - Tissue | ENHERTU (fam-trastuzumab deruxtecan-nxki) BLA 761139 | ERBB2 | ERBB2 Activating Mutations (SNVs And Exon 20 Insertions) |
| 54 | Colorectal cancer - Tissue | Vectibix (panitumumab) BLA 125147 | KRAS and NRAS | KRAS wild-type (absence of mutations in exons 2, 3, and 4) and NRAS wild type (absence of mutations in exons 2, 3, and 4) |
| 55 | Colorectal Cancer - Tissue | Braftovi (encorafenib) NDA 210496 in combination with Erbitux (cetuximab) BLA 125084 | BRAF | V600E |
| 56 | Non-Small Cell Lung Cancer (NSCLC) - Tissue | Gilotrif (afatinib) NDA 201292 | EGFR (HER1) | Exon 19 deletion or exon 21 L858R substitution mutation |
| 57 | Non-Small Cell Lung Cancer (NSCLC) - Tissue | Iressa (gefitinib) NDA 206995 | EGFR (HER1) | Exon 19 deletion or exon 21 L858R substitution mutation |
| 58 | Non-Small Cell Lung Cancer (NSCLC) - Tissue | Gilotrif (afatinib) NDA 201292 | EGFR (HER1) | L861Q, G719X and S7681 |
| 59 | Non-Small Cell Lung Cancer (NSCLC) - Tissue | Vizimpro (dacomitinib) NDA 211288 | EGFR (HER1) | Exon 19 deletion or exon 21 L858R substitution mutation |
| 60 | Urothelial Cancer - Tissue | Balversa (erdafitinib) NDA 212018 | FGFR3 | Exon 7: R248C (c.742C>T), S249C (c.746C>G); exon 10: G370C (c.1108G>T) and Y373C (c.1118A>G); and fusions (FGFR3-TACC3v1 and FGFR3-TACC3v3) |
| 61 | Colorectal Cancer - Tissue | Vectibix (panitumumab) BLA 125147 | KRAS | G12A, G12D, G12R, G12C, G12S, G12V, G13D |
| 62 | Non-Small Cell Lung Cancer (NSCLC) - Tissue | Lumakras (sotorasib) NDA 214665 | KRAS | G12C |
| 63 | Colorectal Cancer - Tissue | Erbitux (cetuximab) BLA 125084 | KRAS | G12A, G12D, G12R, G12C, G12S, G12V, G13D |
| 64 | Colorectal Cancer - Tissue | Erbitux (cetuximab) BLA 125084 | KRAS | KRAS wild-type (absence of mutations in codons 12 and 13) |
| 65 | Non-Small Cell Lung Cancer (NSCLC) - Tissue | Krazati (adagrasib) NDA 216340 | KRAS | KRAS G12C |
| 66 | Breast Cancer - Tissue or Plasma | Piqray (alpelisib) NDA 212526 | PIK3CA | C420R, E542K, E545A, E545D [1635G>T only], E545G, E545K, Q546E, Q546R, H1047L, H1047R, and H1047Y |
| 67 | Melanoma - Tissue | Mekinist (trametinib) NDA 204114 | BRAF | V600E or V600K |
| 68 | Melanoma - Tissue | Tafinlar (dabrafenib) NDA 202806 | BRAF | V600E |
| 69 | Melanoma - Tissue | Braftovi (encorafenib) NDA 210496 in combination with Mektovi (binimetinib) NDA 210498 | BRAF | V600E or V600K |
| 70 | B-cell Chronic Lymphocytic Leukemia - Peripheral Blood | Venclexta (venetoclax) NDA 208573 | TP53 | Deletion chromosome 17p (17p-) |

FIG. 7A

ANALYSIS TARGET REGION

PREDETERMINED
SITE (1 BASE)

REFERENCE
SEQUENCE

X                                    Y

FIRST READ

⇩                    ⇩
DEPTH A          DEPTH B

FIG. 7B

ANALYSIS TARGET REGION

PREDETERMINED SITE
(PLURALITY OF BASES)

REFERENCE
SEQUENCE

X ················· Y

FIRST READ

⇩
DEPTH C

FIG. 8

START

READ OUT RESULT OF ALIGNMENT BETWEEN TUMOR SEQUENCE DATA AND REFERENCE SEQUENCE — S41

OBTAIN DEPTH INFORMATION T AT PREDETERMINED SITE — S42

READ OUT RESULT OF ALIGNMENT BETWEEN NORMAL SEQUENCE DATA AND REFERENCE SEQUENCE DATA — S43

OBTAIN DEPTH INFORMATION N AT PREDETERMINED SITE — S44

STORE DEPTH INFORMATION T AND DEPTH INFORMATION N AT PREDETERMINED SITE — S45

RETURN

FIG. 9

R

TEST REQUEST INFORMATION R1

TEST REQUEST ID : x x x x x

· · · · · · ·

RESULT OF MUTATION DETECTION R2

| Chr | Gene | Pos | CDS | AA | Somatic/Germline | Mutation type |
|---|---|---|---|---|---|---|
| 1 | gene A | 115,252,203 | c.437C>T | A146V | somatic | SNV |
| 7 | gene C | · · · | · · · | · · · | · · · | · · · |
| · | · | · | · | · | · | · |

QUALITY INFORMATION ON NUCLEIC ACID ANALYSIS R3

| Chr | Gene | AA | Pos | Depth T | Determination result T | Depth N | Determination result N | Result of mutation detection |
|---|---|---|---|---|---|---|---|---|
| 1 | gene A | A146V | 115,252,203 | 500 | ✓ | 250 | ✓ | YES |
| 2 | gene B | R1275Q | 29,432,664 | 480 | ✓ | 200 | ✓ | NO |
| 2 | gene B | G1269A | 29,432,682 | 450 | | 210 | ✓ | NO |
| · | · | · | · | · | · | · | · | · |

FIG. 10

```
        ┌─────────────┐
        │    START    │
        └──────┬──────┘
               │
    ┌──────────┴──────────┐
    │ RECEIVE SELECTION OF│   ⌇ S51
    │  PREDETERMINED SITE │
    └──────────┬──────────┘
               │
    ┌──────────┴──────────┐
    │   READ OUT READS    │   ⌇ S52
    └──────────┬──────────┘
               │
    ┌──────────┴──────────┐
    │ OBTAIN RESULT OF    │   ⌇ S53
    │ MUTATION DETECTION  │
    │ IN ANALYSIS TARGET  │
    │      REGION         │
    └──────────┬──────────┘
               │
    ┌──────────┴──────────┐
    │ GENERATE INFORMATION│   ⌇ S54
    │ REGARDING QUALITY OF│
    │ NUCLEIC ACID ANALYSIS│
    │ WITH RESPECT TO     │
    │ PREDETERMINED SITE  │
    └──────────┬──────────┘
               │
    ┌──────────┴──────────┐
    │ OUTPUT TEST RESULT  │   ⌇ S55
    │      REPORT         │
    └──────────┬──────────┘
               │
    ┌──────────┴──────────┐
    │ STORE TEST RESULT   │   ⌇ S56
    │ REPORT INTO         │
    │ STORAGE UNIT        │
    └──────────┬──────────┘
               │
        ┌──────┴──────┐
        │     END     │
        └─────────────┘
```

## FIG. 11A

D1

CATEGORY — D11

SETTING LIST — D12

CDx marker
Driver mutation
Malignancy marker
Prognosis marker
Morbidity risk marker
Onset risk marker
. . .

| Chr | Gene | AA | Pos |
|-----|--------|--------|-------------|
| 1 | gene A | A146V | 115,252,203 |
| 2 | gene B | R1275Q | 29,432,664 |
| 2 | gene B | G1269A | 29,432,682 |
| . | . | . | . |
| . | . | . | . |
| . | . | . | . |

D13 — SET   CANCEL

## FIG. 11B

D2

CATEGORY D21    SUBCATEGORY D22    SETTING LIST D23

CDx marker

JP
US
EP
CN
. . .

| Chr | Gene | AA | Pos |
|-----|--------|--------|-------------|
| 1 | gene A | A146V | 115,252,203 |
| 2 | gene B | R1275Q | 29,432,664 |
| . | . | . | . |
| . | . | . | . |

D24 — SET   CANCEL

FIG. 11C

D3

| CATEGORY D31 | SUBCATEGORY D32 | SETTING LIST D33 |

**CATEGORY** D31

(Biomarker)
CDx marker
Malignancy marker
⋮

(Disease information)
Disease name
Disease type
Driver mutation
Evidence level
Evidence level
Evidence type
⋮

(Drug information)
Presence or absence
of therapeutic drug
Therapeutic drug name
⋮

**SUBCATEGORY** D32

(Evidence
classification)
Lv A
Lv B
Lv C
⋮

**SETTING LIST** D33

| Chr | Gene | AA | Pos |
|-----|--------|--------|------------|
| 2 | gene B | R1275Q | 29,432,664 |
| 2 | gene B | G1269A | 29,432,682 |
| ⋮ | ⋮ | ⋮ | ⋮ |

D34 | SET | CANCEL |

FIG. 11D

D4

**CATEGORY** D41

Discretionary
selection ▼

**SETTING LIST** D42

| Chr | Pos_Start | | Pos_End | |
| | | ~ | | ⊕ ⊖ |
| | | ~ | | ⊕ ⊖ |
| | | ~ | | ⊕ ⊖ |

D43 | SET | CANCEL |

FIG. 12

```
                        ┌──────────┐
                        │  START   │
                        └────┬─────┘
                             │
   S61 ┌──────────────────────────────────────────────┐
       │   ALIGNMENT BETWEEN TUMOR SEQUENCE DATA       │
       │          AND REFERENCE SEQUENCE               │
       └──────────────────────┬───────────────────────┘
                              │
   S62                       ╱╲
              ╱──────────────────────────────╲              No
             ╱   DOES TUMOR SEQUENCE DATA      ╲───────────────┐
             ╲  MISMATCH REFERENCE SEQUENCE?   ╱                │
              ╲──────────────────────────────╱                 │
                            │                                   │
                           Yes                                  │
   S63 ┌──────────────────────────────────────────────┐        │
       │  SEARCH MUTATION INFORMATION DATABASE ON      │        │
       │        THE BASIS OF DETECTED MUTATION         │        │
       └──────────────────────┬───────────────────────┘        │
                              │                                 │
   S64 ┌──────────────────────────────────────────────┐        │
       │      PROVIDE ANNOTATION TO DETECTED           │        │
       │  MUTATION ON THE BASIS OF SEARCH RESULT       │        │
       └──────────────────────┬───────────────────────┘        │
                              │◄────────────────────────────────┘
                        ┌──────────┐
                        │  RETURN  │
                        └──────────┘
```

FIG. 13

```
          ┌─────────────┐
          │    START    │
          └──────┬──────┘
                 │
   ┌─────────────┴──────────────────┐
   │  READ OUT RESULT OF ALIGNMENT  │    S71
   │  BETWEEN TUMOR SEQUENCE DATA    │
   │    AND REFERENCE SEQUENCE       │
   └─────────────┬──────────────────┘
                 │
   ┌─────────────┴──────────────────┐
   │  OBTAIN DEPTH INFORMATION T AT  │   S72
   │      PREDETERMINED SITE         │
   └─────────────┬──────────────────┘
                 │
   ┌─────────────┴──────────────────┐
   │  STORE DEPTH INFORMATION T AT   │   S73
   │      PREDETERMINED SITE         │
   └─────────────┬──────────────────┘
                 │
                 ▼
          ┌─────────────┐
          │   RETURN    │
          └─────────────┘
```

Europäisches
Patentamt

European
Patent Office

Office européen
des brevets

## EUROPEAN SEARCH REPORT

Application Number

EP 24 18 4272

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 588 508 A1 (SYSMEX CORP [JP]) 1 January 2020 (2020-01-01) * abstract * * paragraphs [0002], [0010], [0021], [0053], [0089], [0103], [0171], [0190], [0242]; claim 1 * | 1-19 | INV. G16B30/00 G16B20/50 |
| X | US 2020/258601 A1 (LAU DENISE [US]) 13 August 2020 (2020-08-13) * abstract * * paragraphs [0056], [0234], [0240], [0335], [0367], [0369], [0390]; claim 1 * | 1-19 | |

-----

-----

TECHNICAL FIELDS
SEARCHED        (IPC)

G16B

The present search report has been drawn up for all claims

| Place of search | Date of completion of the search | Examiner |
|---|---|---|
| Munich | 14 November 2024 | Schmitt, Constanze |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 18 4272

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2024

| Patent document cited in search report | | Publication date | Patent family member(s) | | Publication date |
|---|---|---|---|---|---|
| EP 3588508 | A1 | 01-01-2020 | CN | 110660449 A | 07-01-2020 |
| | | | EP | 3588508 A1 | 01-01-2020 |
| | | | JP | 6920251 B2 | 18-08-2021 |
| | | | JP | 7148681 B2 | 05-10-2022 |
| | | | JP | 7399238 B2 | 15-12-2023 |
| | | | JP | 7577822 B2 | 05-11-2024 |
| | | | JP | 2020000198 A | 09-01-2020 |
| | | | JP | 2021176096 A | 04-11-2021 |
| | | | JP | 2022180553 A | 06-12-2022 |
| | | | JP | 2024026278 A | 28-02-2024 |
| | | | US | 2020020418 A1 | 16-01-2020 |
| US 2020258601 | A1 | 13-08-2020 | EP | 4104175 A1 | 21-12-2022 |
| | | | US | 2020258601 A1 | 13-08-2020 |
| | | | WO | 2021163233 A1 | 19-08-2021 |

For more details about this annex : see Official Journal of the European Patent Office, No. 12/82

**REFERENCES CITED IN THE DESCRIPTION**

**Patent documents cited in the description**

• JP 2023108392 A